# EUROPEAN PATENT APPLICATION

(11) **EP 3 611 510 A1**
(43) Date of publication of application: **19.02.2020**
(21) Application number: 19202544.3
(22) Date of filing: 27.07.2010
(51) Int. Cl.: G01N 35/02, G01N 1/28, G01N 33/48, G01N 21/00

(54) **ASSAY APPARATUSES, CONSUMABLES AND METHODS**

(30) Priority: 27.07.2009 US 27187409 P
(62) Divisional of application: 10806884.2
(71) Applicant: Meso Scale Technologies, LLC, Gaithersburg, MD 20877 (US)
(72) Inventor: CHAMBERLIN, Ian, Burtonsville, MD Maryland 20866 (US); CLINTON, Charles M., Clarksburg, MD Maryland 20871 (US); GLEZER, Eli N., Chevy Chase, MD Maryland 20815 (US); JEFFREY-COKER, Bandele, Darnestown, MD Maryland 20878 (US); KOCHAR, Manish, Rockville, MD Maryland 20853 (US); KOVACS, Sandor, Middletown, DE Delaware 19709 (US); LE, Dt, Beltsville, MD Maryland 20705 (US); LEIMKUEHLER, Aaron, Pittsburgh, PA Pennsylvania 15241 (US); PINCKNEY, Greg, Myersville, MD Maryland 21773 (US); ROTH, Kristian, Germantown, MD Maryland 20874 (US); SIGAL, George, Rockville, MD Maryland 20853 (US); YIN, Fei, North Potomac, MD Maryland 20878 (US)
(74) Representative: Modiano, Gabriella Diana

(57) **Abstract**

We describe apparatuses, method, reagents, and kits for conducting assays as well as process for their preparation. They are particularly well suited for conducting automated sampling, sample preparation, and analysis in a multi-well plate assay format. For example, they may be used for automated analysis of liquid samples in a clinical point of care setting.

## Description

### CROSS REFERENCE TO RELATED APPLICATION

This application claims the benefit of U.S. Provisional Application No. 61/271,874 filed on July 27, 2009, the entire contents of which are incorporated herein by reference.

### STATEMENT REGARDING FEDERALLY-SPONSORED RESEARCH

This invention was made with federal support under HHS 200-2008-25451 awarded by the Department of Health and Human Services and the Centers for Disease Control. The U.S. government has certain rights in the invention.

### FIELD OF THE INVENTION

The invention relates to apparatuses, consumables, methods, and kits for conducting assays. Certain embodiments of the invention may be used for conducting automated sampling, sample preparation, and/or sample analysis in a multi-well plate assay format.

### BACKGROUND OF THE INVENTION

Numerous methods and apparatus have been developed for conducting chemical, biochemical, and/or biological assays. These methods and apparatus are essential in a variety of applications including medical diagnostics, food and beverage testing, environmental monitoring, manufacturing quality control, drug discovery, and basic scientific research.

Multi-well assay plates (also known as microtiter plates or microplates) have become a standard format for processing and analysis of multiple samples. Multi-well assay plates can take a variety of forms, sizes, and shapes. For convenience, some standards have appeared for instrumentation used to process samples for high-throughput assays. Multi-well assay plates typically are made in standard sizes and shapes, and have standard arrangements of wells. Arrangements of wells include those found in 96-well plates (12 x 8 array of wells), 384-well plates (24 x16 array of wells), and 1536-well plates (48 x 32 array of wells). The Society for Biomolecular Screening has published recommended microplate specifications for a variety of plate formats (see http://www.sbsonline.org).

A variety of apparatuses are available for conducting assay measurements in multi-well plates including instruments that measure changes in optical absorbance, emission of luminescence (e.g., fluorescence, phosphorescence, chemiluminescence, and electrochemiluminescence (ECL)), emission of radiation, changes in light scattering, and changes in a magnetic field. U.S. Patent Application Publications 2004/0022677 and 2005/0052646 describe solutions that are useful for carrying out singleplex and multiplex ECL assays in a multi-well plate format. They include plates that comprise a plate top with through-holes that form the walls of the wells and a plate bottom that is sealed against the plate top to form the bottom of the wells. The plate bottom has patterned conductive layers that provide the wells with electrode surfaces that act as both solid phase supports for binding reactions as well as electrodes for inducing ECL. The conductive layers may also include electrical contacts for applying electrical energy to the electrode surfaces. Reference is also made to U.S. Application Serial No. 11/642,968.

Despite such known methods and apparatuses for conducting assays, improved apparatuses, apparatus', methods, reagents, and kits for conducting automated sampling, sample preparation, and/or sample analysis in a multi-well plate assay format are needed.

### SUMMARY OF THE INVENTION

Therefore, the present invention provides a kit for conducting luminescence assays in multi-well plates, the kit comprising:
(a) a multi-well assay test plate comprising a plurality of assay wells for the assay;
(b) an auxiliary plate comprising a plurality of auxiliary wells, the auxiliary well comprising dry assay reagents for use in the assay with the assay test plate. A well of the test plate may comprise a plurality of distinct assay domains, at least two of the domains comprising reagents for measuring different analytes. In one embodiment, the auxiliary plate comprises an identifier comprising assay information used to identify an element selected from the group consisting of (i) the auxiliary plate, (ii) one or more auxiliary wells within the auxiliary plate, (iii) a reagent and/or sample that has been or will be used with the auxiliary plate, (iv) the test plate, (v) one or more wells within the test plate, (vi) a reagent and/or sample that has been or will be used with the test plate, and (vii) combinations thereof. The test plate information may identify a test plate for use with an auxiliary plate. In one embodiment, the test plate information comprises test plate lot information, e.g., a test plate identification number.

In one embodiment, the assay test plate comprises an identifier comprising assay information used to identify an element selected from the group consisting of (i) the auxiliary plate, (ii) one or more auxiliary wells within the auxiliary plate, (iii) a reagent and/or sample that has been or will be used with the auxiliary plate, (iv) the test plate, (v) one or more wells within the test plate, (vi) a reagent and/or sample that has been or will be used with the test plate, and (vii) combinations thereof. In this embodiment, the identifier may comprise auxiliary plate information identifying an auxiliary plate for use with a test plate, e.g., auxiliary plate lot information, e.g., an auxiliary plate identification number.

The plurality of auxiliary wells in the auxiliary plate may be a multiple of the number of assay wells in the assay test plate, e.g., twice or four times as many auxiliary wells as assay wells in the assay test plate. In one embodiment, the auxiliary plate further comprises a set of auxiliary wells, the set comprising adjacent auxiliary wells, wherein the set of auxiliary wells comprises reagents for an assay in a well of the assay test plate. The set may comprise four adjacent auxiliary wells, e.g., arranged in a square and/or in a row. The set may further comprise a dilution well and/or pre-treated beads (which may be magnetic and/or may comprise a coating selected from the group consisting of streptavidin, biotin, and avidin, and one or more reagents in the set of auxiliary wells comprise a binding partner of the coating).

Still further, the invention contemplates a kit including an auxiliary plate wherein at least one auxiliary well of the set comprises desiccant and the auxiliary plate comprises a seal. In this embodiment, the at least one auxiliary well of the set may be connected to an additional auxiliary well of the set via an air passage. The at least one auxiliary well of the set may be connected to all auxiliary wells of the set via the air passage.

The invention further provides an auxiliary plate comprising a plurality of auxiliary wells, the auxiliary wells comprising dry assay reagents for use in an assay with a corresponding assay test plate. The auxiliary plate may comprise an identifier comprising assay information used to identify an element selected from the group consisting of (i) the auxiliary plate, (ii) one or more auxiliary wells within the auxiliary plate, (iii) a reagent and/or sample that has been or will be used with the auxiliary plate, (iv) the test plate, (v) one or more wells within the test plate, (vi) a reagent and/or sample that has been or will be used with the test plate, and (vii) combinations thereof. The identifier may further include test plate information identifying a test plate for use with the auxiliary plate, e.g., the test plate information comprises test plate lot information, e.g., a test plate identification number. The plurality of auxiliary wells in the auxiliary plate may be a multiple of the number of wells in the assay test plate, e.g., twice or four times as many auxiliary wells as wells in the assay test plate. In one embodiment, the auxiliary plate further comprises a set of auxiliary wells, the set comprising adjacent auxiliary wells, wherein the set of auxiliary wells comprises reagents for an assay in a well of the assay test plate. The set may comprise four adjacent auxiliary well, e.g., arranged in a square and/or in a row. The set may include a dilution well and/or pre-treated beads (which may be magnetic and/or may comprise a coating selected from the group consisting of streptavidin, biotin, and avidin, and one or more reagents in the set of auxiliary wells comprise a binding partner of the coating).

In one embodiment, at least one auxiliary well of the set comprises desiccant and the auxiliary plate comprises a seal, and optionally, at least one auxiliary well of the set is connected to an additional auxiliary well of the set via an air passage. In a further embodiment, at least one auxiliary well of the set is connected to all auxiliary wells of the set via the air passage.

The invention also provides an apparatus for conducting a measurement in a multi-well assay test plate, the apparatus comprising
(a) a subassembly capable of supporting and translating the test plate to one or more components of the apparatus; and
(b) an auxiliary plate subassembly, wherein the auxiliary plate comprises a plurality of auxiliary wells comprising dry assay reagents for use in an assay with the test plate.

The apparatus of the invention may further include a pipettor subassembly that delivers sample and/or reagent to and from a well of the test plate and/or an auxiliary well of the auxiliary plate. In one embodiment, the pipettor subassembly comprises a component selected from the group consisting of a pump, a plate piercing probe, a pipetting probe and an ultrasonic sensor.

Subassembly (a) of the apparatus may include one or more of the following components: a plate introduction aperture and a plate translation stage; a plate stacker adjacent to the plate introduction aperture; a plate elevator comprising a plate lifting platform that can be raised and lowered onto the plate translation stage; an input plate introduction aperture comprising an input plate stacker and an output plate introduction aperture comprising an output plate stacker. The plate stacker may be able to accommodate more than one test plate. In one embodiment, the subassembly (a) is or includes a light-tight enclosure and the plate introduction aperture comprises a sliding light-tight door. Additional components include but are not limited to a thermoelectric heater/cooler, a desiccant chamber, and an identifier controller, an imaging system mounted to an imaging aperture in the light-tight enclosure. In one embodiment, the plate translation stage is configured to position a well of a test plate in proximity to one or more components of the subassembly selected from the group consisting of the plate elevator, a well-wash subassembly, and an imaging system. The well-wash subassembly may comprise a seal removal tool, a well-wash head, a wash station, and fluidic connectors to a liquid reagent subassembly. The well-wash head may include a pipetting probe and a pipetting translation stage for translating the pipetting probe in a vertical direction. In one embodiment, the pipetting probe comprises a dispensing tube and a plurality of aspiration tubes.

The auxiliary plate subassembly of the apparatus of the invention may comprise an auxiliary plate introduction aperture and a plate support. The auxiliary plate subassembly may further include a housing comprising two or more compartments, each compartment comprising the auxiliary plate introduction aperture and the plate support. In one embodiment, the compartments comprise a component selected from the group consisting of an identifier controller, a thermoelectric heater/cooler, and a desiccant chamber.

Also provided is a method for conducting a measurement in a multi-well assay test plate, the method comprising the steps of:
(a) dispensing sample and/or reagent into an auxiliary well of an auxiliary plate, the auxiliary plate comprising a plurality of auxiliary wells, the plurality of auxiliary wells comprising dry assay reagents for use in an assay with the test plate; and
(b) transferring sample and/or reagent from the auxiliary well to a well of a the assay test plate.

In one embodiment, dispensing step (a) comprises pre-treating the sample and/or reagent in the auxiliary well. Still further, transferring step (b) may comprise dispensing pre-treated sample and/or reagent from the auxiliary well to the well of the test plate.

In a further embodiment, the assay test plate is supported on a plate translation stage and the method comprises translating the test plate via the plate translation stage to one or more components of the apparatus. And in this embodiment, the method may further include placing the assay test plate through a plate introduction aperture onto a plate stacker adjacent the plate translation stage, and optionally, lowering the assay test plate from the plate stacker to the plate translation stage.

Still further, the method of the present invention further comprises (i) placing the assay test plate through an input plate introduction aperture comprising an input plate stacker, (ii) lowering the assay test plate from the input plate stacker to the plate translation stage, (iii) translating the assay test plate to one or more components of the apparatus to conduct the measurement, (iv) raising the assay test plate from the plate translation stage to an output plate stacker, and (v) removing the assay test plate from an output plate introduction aperture.

Moreover, the method may include repeating steps (a) and (b) in an additional auxiliary well of the auxiliary plate and an additional test well of the test plate.

Also provided is a method for conducting a measurement in a multi-well assay test plate, the method comprising the steps of:
(a) dispensing sample and/or reagent into an auxiliary well of a set of auxiliary wells in an auxiliary plate, the auxiliary plate comprising a plurality of auxiliary wells, the plurality of auxiliary wells comprising dry assay reagents for use in an assay with the test plate;
(b) pre-treating the sample and/or reagent in one or more auxiliary wells of the
   set;
(c) dispensing pre-treated sample and/or reagent from the one or more auxiliary wells of the set to a well of a the assay test plate;
(d) repeating steps (a)-(c) with an additional sample and reagents in an additional set of auxiliary wells in the auxiliary plate, and an additional well of the test plate;
(e) ejecting a used auxiliary plate;
(f) repeating steps (a)-(c) with an additional auxiliary plate; and
(g) ejecting a used assay test plate.

Finally, the invention provides a well-wash subassembly comprising a multi-tube array including a central dispensing tube element surrounded by a plurality of aspiration tube elements. The multi-tube array may include at least two dispensing tube elements at the center of the array, e.g., the dispensing tube elements comprise an independent fluid channel for buffers and/or diluents used during an assay. In one embodiment, the aspiration tube elements surround the dispensing tube elements and the aspiration tube elements are positioned to align with the outer portions of a well bottom of a multi-well test plate. The aspiration tube elements are independently connected to dedicated fluidic lines. In one embodiment, the multi-tube array comprises at least four aspiration tube elements.

### BRIEF DESCRIPTION OF DRAWINGS

Fig. 1(a) is a drawing of one embodiment of an apparatus of the invention.
Fig. 1(b) shows a view of an apparatus of the invention that includes an optional housing (10) and a user interface (20).
Fig. 2 is a drawing of a sample rack subassembly of an apparatus of the invention.
Fig. 2b is a schematic top view of a part of a sample rack subassembly of an apparatus of the invention.
Fig. 2c is a schematic top view of a sample rack subassembly of an apparatus of the invention.
Fig. 3 shows the light-tight enclosure of an apparatus of the invention.
Fig. 4(a) is a drawing of an assay test plate used in an apparatus of the invention.
Fig. 4(b) is an expanded view of one well of an assay test plate.
Fig. 5(a) shows the auxiliary plate subassembly of an apparatus of the invention.
Fig. 5(b) is a drawing of an auxiliary plate used in the auxiliary plate subassembly of an apparatus of the invention.
Figs. 5(c)-5(h) show alternate views of a set of auxiliary wells in the auxiliary plate depicted Figure 5(b).
Fig. 6(a) shows the pipettor subassembly used in an apparatus of the invention.
Figs. 6(b)-(e) show a pipetting tip sensor in the pipettor subassembly. Figs. 6(b)-(c) show the side and front views, respectively, of a pipetting tip sensor including a reflective sensor, wherein the pipetting probe does not include a pipetting tip. Figs. 6(d)-(e) show the side and front views, respectively, of the pipetting tip sensor including a reflective sensor, wherein the pipetting probe includes a pipetting tip.
Fig. 7 shows the disposable tip/waste compartment used in an apparatus of the invention.
Fig. 8 shows the liquid reagent subassembly used in an apparatus of the invention.
Fig. 9 shows the well-wash subassembly used in an apparatus of the invention.

### DESCRIPTION OF SPECIFIC EMBODIMENTS OF THE INVENTION

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The articles "a" and "an" are used herein to refer to one or to more than one (i.e., to at least one) of the grammatical object of the article. By way of example, "an element" means one element or more than one element.

Described herein are apparatuses and associated assay consumables for conducting assays in a multi-well plate format that have one or more of the following desirable attributes: (i) high sensitivity, (ii) large dynamic range, (iii) small size and weight, (iv) array-based multiplexing capability, (v) automated operation (including sample and/or reagent delivery); (vi) ability to simultaneously handle multiple plates, (vii) ability to store and access reagents in auxiliary plates, and (viii) ability to handle sealed plates. Also described are assay consumables that are useful in such an apparatus, and methods for using such an apparatus and components. The assay consumables used in the apparatus includes multi-well assay test plates, auxiliary plates (and kits including a test plate and a corresponding auxiliary plate), and liquid reagents. The assay apparatuses and associated consumables are particularly well suited for, although not limited to, use for autonomous analysis of environmental, clinical, or food samples. The apparatus and methods may be used with a variety of assay detection techniques involving the measurement of one or more detectable signals. Some of them are suitable for ECL measurements and, in particular, embodiments that are suitable for use with multi-well plates with integrated electrodes (and assay methods using these plates) such as those described in U.S. Publications 2004/0022677 and 2005/0052646 and U.S. Application 11/642,970.

Therefore, in one embodiment, the invention provides an apparatus for conducting a measurement in a multi-well assay test plate, the apparatus comprising
(a) a subassembly capable of supporting and translating the test plate to one or more components of the apparatus; and
b) an auxiliary plate subassembly capable of holding an auxiliary plate for holding and/or preparing samples and/or reagents for analysis in the test plate. The auxiliary plate comprises a plurality of auxiliary wells which may comprise assay reagents for use in an assay with the test plate. The assay reagents may be provided in liquid or dry form. In one embodiment, the reagents are provided in dry form in the auxiliary plate.

The apparatus of the invention may further include a pipettor subassembly that delivers sample and/or reagent to and from a well of the test plate and/or an auxiliary well of the auxiliary plate. The pipettor subassembly comprises one or more pipetting probes and may also include one or more components selected from the group consisting of a pump, a plate piercing probe, and an ultrasonic sensor.

The subassembly capable of supporting and translating the test plate comprises a plate translation stage and may further comprise an enclosure in which the plate translation stage is held and which has a plate introduction aperture through which a plate may be placed or removed from the plate translation stage. The enclosure may also include a plate stacker adjacent to the plate introduction aperture. In one embodiment, the subassembly includes a plate elevator comprising a plate lifting platform that can be raised and lowered to transfer plates between a plate stacker and the plate translation stage. In one specific embodiment, the subassembly comprises an enclosure having an enclosure top with input and output plate apertures, the enclosure comprising (i) an input plate elevator and an output plate elevator, each with a plate lifting platform that can be raised and lowered; (ii) a plate translation stage in the enclosure which can support one or more assay plates and translate the plates in one or more horizontal directions within the enclosure, the plate translation stage having openings to allow plate elevators positioned beneath a plate to access and lift the plate and (iii) input and output plate stackers mounted on the enclosure top above the input and output apertures, respectively, the plate stackers being configured to receive or deliver plates to the input and output plate elevators, respectively. The plate translation stage is configured to position a test plate so that it can be accessed by the plate elevators and/or such that wells of the test plate can be placed in proximity to and/or accessed by one or more additional components of the apparatus selected from the group consisting of a well-wash subassembly, a pipetting probe and a detection subsystem.

The subassembly enclosure may further comprise a sliding door configured to close the plate introduction apertures, as well as one or more of the following components: a thermoelectric heater/cooler, a desiccant chamber, an identifier controller, an imaging apparatus mounted to an imaging aperture in the light-tight enclosure. Optionally, the sliding door provides a light-tight seal allowing the sub-assembly enclosure to be used as a light-tight enclosure for luminescence measurements.

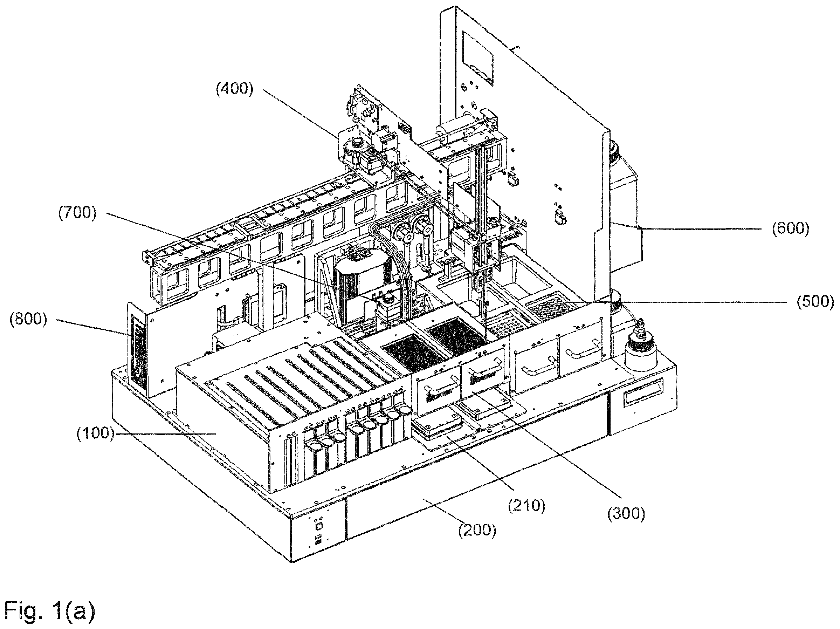

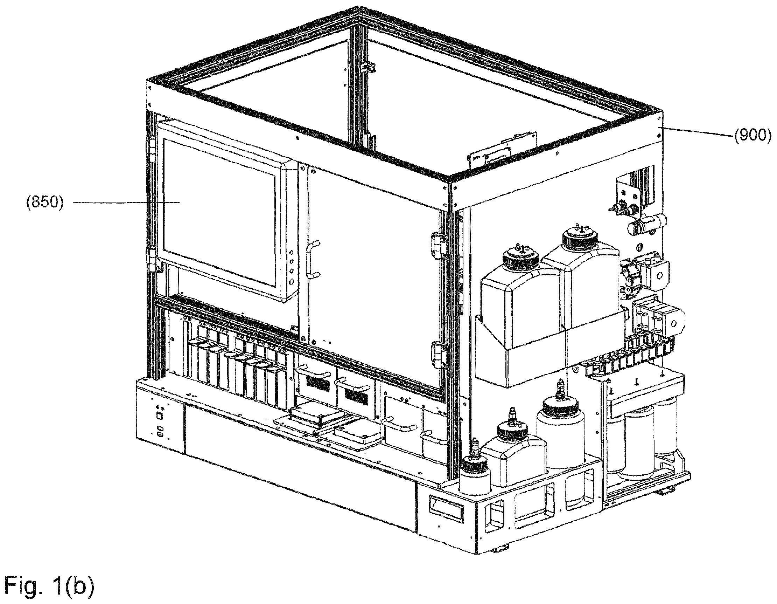

One embodiment of the apparatus of the invention is shown in Fig. 1(a). The apparatus includes the following components: (i) a sample rack subassembly (100); (ii) a light-tight enclosure (200); (iii) an auxiliary plate subassembly (300); (iv) a pipettor subassembly (400); (v) a pipetting tip storage/disposal compartment (500); (vi) a liquid reagent subassembly (600); (vii) a well-wash subassembly (700); and (viii) a power supply (800). The apparatus is also attached to a computer through a user interface (850) (shown in an optional configuration in Fig. 1(b)). These components are described in more detail below. This apparatus of the present invention enables fully automated random access analysis of samples using array-based multiplexed multi-well plate consumables. The apparatus achieves enhanced sensitivity and high sample throughput. It may be adapted for use with any of a variety of detection techniques, e.g., changes in optical absorbance, emission of luminescence or radiation, changes in light scattering and/or changes in a magnetic field. In one embodiment, the apparatus is configured to detect the emission of luminescence, e.g., fluorescence, phosphorescence, chemiluminescence and ECL. In a particular embodiment, the apparatus is configured to detect ECL. All the biological reagents required for an assay are provided in the apparatus, thus minimizing the consumable and reagent requirements for the apparatus.

The apparatus may be used for singleplex measurements or it may be configured to enable multiplex measurements. The multiplexing capability of the apparatus provides many advantages, including but not limited to, realizing the maximum amount of information per measurement (simultaneous multiple tests per sample), minimal sample consumption (full sample characterization using a single sample volume), lower consumable costs, simplified assay protocols and minimal user manipulation, the ability to expand assay menus, and the ability to simultaneously carry out control assays.

The various components described herein may be conventional components such as those known in the art. Alternatively, the apparatus may employ specific components as described herein. Furthermore, the apparatus may further comprise electronic components for controlling operation of the apparatus or individual components including, e.g., operating motorized mechanical apparatus, and triggering and/or analyzing luminescence signals. Fig. 1(b) shows a view of the apparatus that includes an optional housing (900) and a user interface (850).

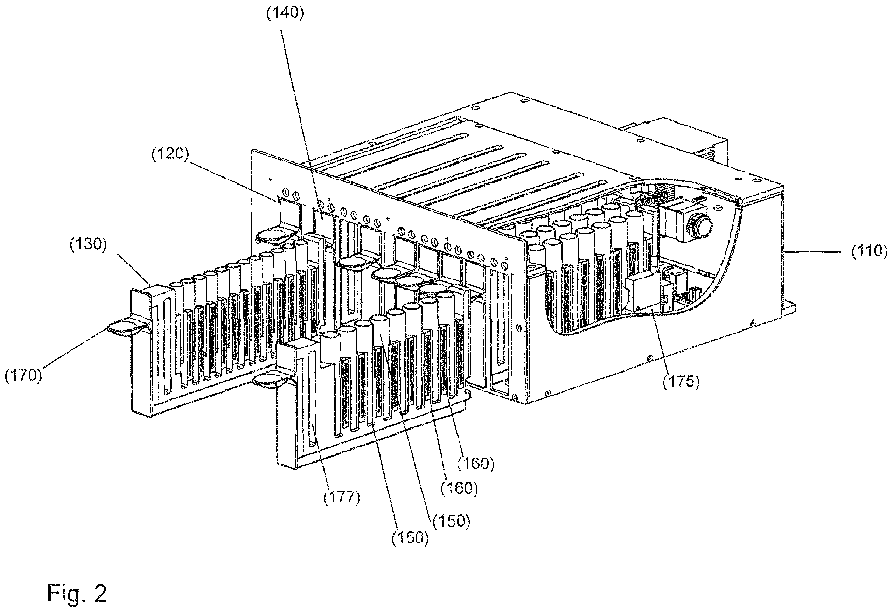

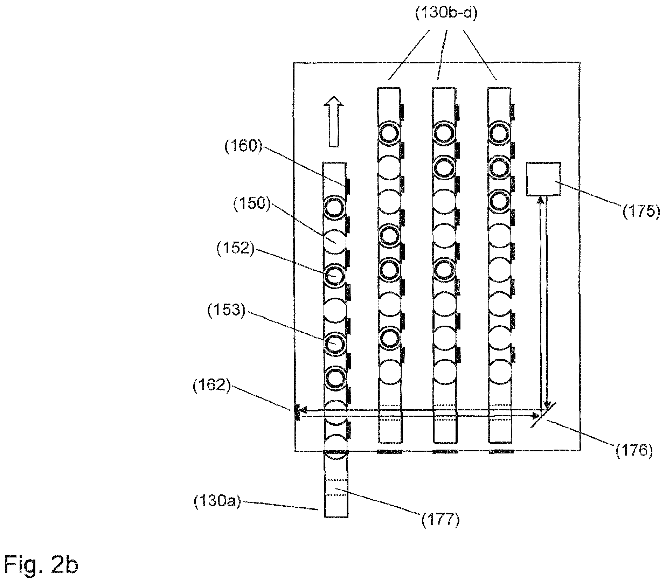

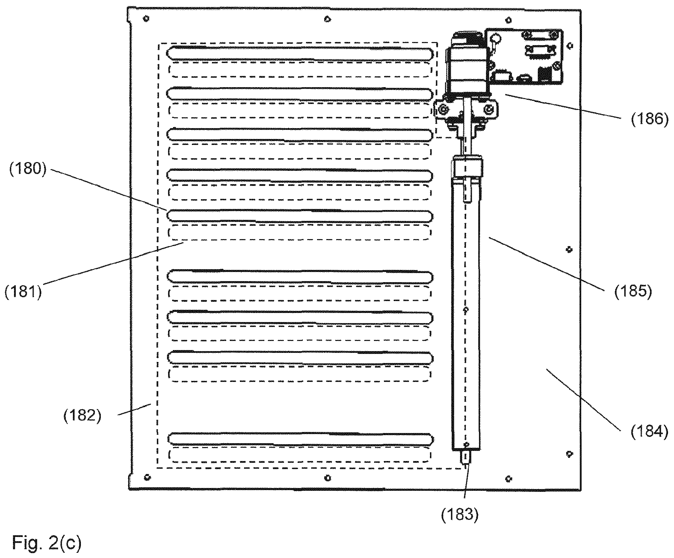

A detailed view of the sample rack subassembly is depicted in Figure 2. The sample rack subassembly includes a housing (110) with a plurality of individual sample rack compartments (120), each one capable of accommodating a sample rack (130). Each sample rack compartment may optionally include a door (140) through which a sample rack is inserted. Each sample rack includes a plurality of sample tube positions (150) separated by a spacer (160). Each sample rack may optionally include a handle (170) and a track mated to a track within the sample rack compartment (not shown) to facilitate insertion of the sample rack into the sample rack compartment. The sample tube positions can be configured to accommodate any dimension sample tube. In one embodiment, the sample tubes are standard 13 mm diameter test tubes, but the skilled artisan will recognize that the sample tube positions are readily adjusted to accommodate any geometry sample tube.

Each sample tube rack includes an identifier that is used to identify the sample or samples in the rack or to identify the rack itself. The identifier, as described below, may be, e.g., a bar code, an EEPROM, or an RFID. In one embodiment, the identifier is a bar code. The apparatus is configured to read identifiers on sample tubes placed in a sample tube rack. Additional identifiers may be placed between or behind sample tube positions to aid in unambiguously identifying the position of specific sample tubes in the tube rack and/or to identify which positions in the sample tube rack hold sample tubes. In one embodiment, the sample tubes are labeled with a bar code to identify the sample in the tube. Still further, the sample tube rack may also include a bar code on each spacer in the rack. The spacer bar codes may be used by the instrument to identify the tube rack and to identify the sample tube position between two spacers. Still further, the sample tube rack may include a bar code behind a sample tube position (relative to the bar code reader) to determine whether a sample tube is present in a given position, e.g., if the tube is present, the bar code is obstructed from view and cannot be read, but if a tube is not present, the bar code is readable. Additionally, the rack itself may include an additional bar code that is used to identify the rack. The sample rack compartment also includes an identifier controller to read and process the data stored to the identifier. For example, if the identifier is a bar code, the identifier controller is a bar code reader. The rack compartment may include two or more bar code readers to accurately read each identifier over the entire span of the sample rack. In one embodiment, the identifiers are read by the bar code reader as the rack is inserted into the compartment. Alternatively, the bar codes are read by the bar code reader after the rack is completely inserted into the compartment, e.g., by moving the reader to scan across the rack. In another embodiment, the user inputs sample and/or rack specific information (either manually or by uploading such information from an external user computer and/or network) into the user interface and the instrument associates that information with each sample and/or rack.

Figure 2b shows a schematic top view (not to scale) of a part of the sample rack subassembly of Figure 2. This part holds four sample tube racks; three racks are fully inserted in the subassembly - racks (130b-d) - and one - rack (130a) is shown in the process of being inserted. The sample tube positions (150) may hold tubes without bar codes (152) or with barcodes (153), where bar codes are indicated as gray bars. The racks also have bar codes on spacer positions (160) before each sample tube position. A bar code reader (175) directs a light source via a mirror (176) to scan bar codes as they are inserted into the sub-assembly enclosure. Apertures (177) in each rack are located just inside the enclosure (when the rack is fully inserted) and allow the light beam (shown as arrows in the figure) to pass through any racks positioned between the reader and a new rack that is in the process of being inserted. The sample tube positions are slotted to allow the light to pass through these positions if no tube is present. As the light source scans a sample tube position, one of three outcomes may result: (i) the reader may read a bar code on a sample tube and thereby identify the tube in that position, (ii) the light source may be blocked by a tube but not read a bar code, identifying that position as holding an non-bar coded tube or a tube with an incorrectly positioned or illegible bar code, or (iii) the light source may pass through the slot and read an "empty position" bar code (162) on the enclosure that identifies the position as being empty (as shown in the Figure). A sample rack subassembly may have multiple sample rack units, such as the one shown in Figure 2b. In operation, when a sample rack compartment is available for insertion of a sample rack (which may be indicated through indicator lights, through unlocking of the compartment door, the instrument GUI, etc.), the bar code reader continuously scans bar codes as the rack is inserted, optionally, until rack seating sensors indicate that the rack is fully inserted, thereby identifying the rack type and the number of tube positions by looking at the spacer bar codes and identifying the contents in each tube position (i.e., empty, non-labeled tube or bar-coded tube) by examining the bar codes (if any) between spacer positions.

Fig. 2c shows a bottom schematic view of the enclosure top of the sample rack subassembly of Fig. 2. Fig. 2c is shown from the perspective of the inside of the sample rack subassembly looking up to the top of the subassembly (184). The top of the subassembly is equipped with a sliding door (dotted line) (182), within which a linear guide access slot is provided. A linear guide (183) driven by a motor (186) is included within the subassembly. The linear guide can access the sliding door (182) and provide free sliding motion within the subassembly in one direction. The subassembly top includes a subassembly sample access slot (180) and the sliding door (182) includes a sample access slot (181). When the sample access slots in the sliding door and the enclosure top are aligned, the pipettor subassembly can access the sample tubes in a given rack and by misaligning the access slots (180 and 181) the enclosure top can be is sealed to maintain the samples tubes in an appropriate temperature and humidity enclosed environment.

In one embodiment, the sample rack subassembly includes a heater/cooling mechanism (e.g., a thermoelectric heater/cooler) to maintain the sample temperature at a desired temperature, as needed.

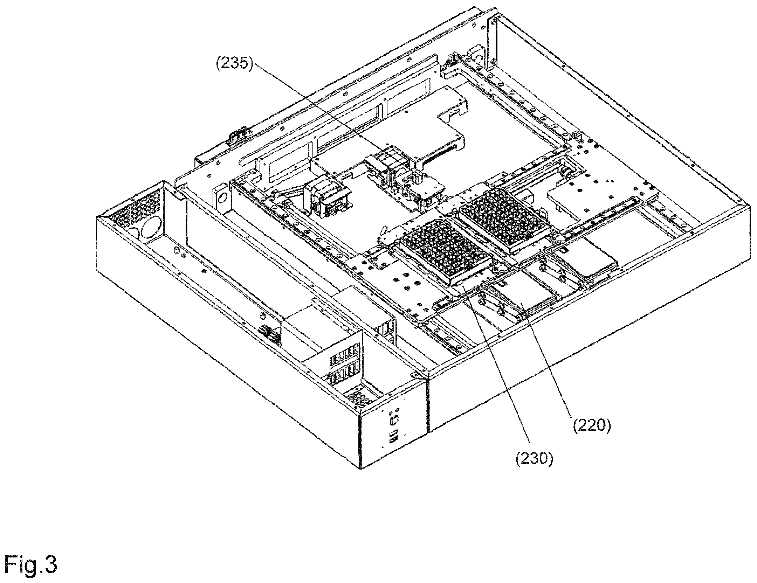

In one embodiment, the apparatus conducts luminescence assays in multi-well plates and the apparatus includes a light-tight enclosure (Fig. 3) that provides a light-free environment in which luminescence measurements may be carried out. The enclosure also includes an enclosure top (not shown) having at least two plate introduction apertures (not shown) through which test plates may be placed onto or removed from a plate stacker (manually or mechanically) - see, plate stacker (210) in Fig. 1(a). In one embodiment, the enclosure includes, defined into the top of the enclosure, (i) an input plate introduction aperture comprising an input plate stacker and (ii) an output plate introduction aperture comprising an output plate stacker, and each plate stacker may accommodate more than one test plate (See Fig. 1(a)). The enclosure top includes a fixed top and a sliding light-tight door (not shown) adjacent to and under the fixed top to seal the plate introduction apertures from environmental light prior to carrying out luminescence measurements. The fixed top and sliding door have small plate access apertures to allow instrument components outside the enclosure (e.g., pipetting probes, plate piercing tools, well washing probes, etc.) to access plates in the enclosure. The sliding door has a plurality of defined positions for different operations, e.g., (i) a fully open position for loading and unloading plates to and from the plate translation stage; (ii) one or more intermediate positions (or "tool access" positions) in which the access apertures for a specific instrument component in the fixed top and the sliding door are aligned such that the component can access plates in the enclosure, and (iii) a closed position in which the plate introduction aperture is sealed from external light and the access apertures are also sealed from external light (i.e., because the apertures in the fixed top and the sliding door are out of alignment).

The enclosure includes one or more plate elevators (220) having plate lifting platforms that can be used to lower plates from a plate stacker onto a plate translation stage (230) or raise them back to a plate stacker (using latches in the stacker to hold or release plates as necessary). The plate translation stage (230) that has horizontal axis of motion for translating a test plate horizontally in the enclosure to zones where specific assay processing and/or detection steps are carried out, e.g., to one or more of an imaging aperture and a component of a well-wash subassembly. In the specific embodiment shown in the figure, two axis of motion (X and Y) are provided by mounting the stage on an X-Y translation table. Motors coupled to the axis of motion allow for automated movement of plates on the table. In one embodiment, the enclosure includes an identifier controller that reads, writes and/or erases assay information to and/or from an identifier associated with a test plate in the enclosure. For example, the light-tight enclosure may include an optical path for a bar code reader to read bar codes on plates placed on the plate translation stage. Alternatively, the test plates may comprise an EEPROM or an RFID and the enclosure includes an identifier controller suitable for communicating with each of these identifiers. In addition, the light-tight enclosure may include heaters and/or coolers (e.g., a thermoelectric heater/cooler) and/or desiccant chamber to maintain the light-tight enclosure under controlled temperature and/or humidity.

An imaging apparatus is mounted on an imaging aperture in the fixed top of the light-tight enclosure and can image luminescence from test plates in the enclosure. The imaging apparatus includes a camera mounted on the top of the light-tight enclosure via a camera bracket. A lens coupled to the camera is used to provide a focused image of luminescence generated from test plates in the enclosure. A diaphragm sealed to the lens and an aperture in the top of the enclosure and allows the imaging apparatus to image light from the enclosure while maintaining the enclosure in a light-tight environment protected from environmental light. Suitable cameras for use in the imaging apparatus include, but are not limited to, conventional cameras such as film cameras, CCD cameras, CMOS cameras, and the like. CCD cameras may be cooled to lower electronic noise. The lens is a high numerical aperture lens which may be made from glass or injection-molded plastic. The imaging apparatus may be used to image one well or multiple wells of a test plate at a time. The light collection efficiency for imaging light from a single well is higher than for imaging a group of wells due to the closer match in the size of the CCD chip and the area being imaged. The reduced size of the imaged area and the increase in collection efficiency allows for the use of small inexpensive CCD cameras and lenses while maintaining high sensitivity in detection. Particularly advantageous, for their low cost and size, is the use of non-cooled cameras or cameras with minimal cooling (preferably to about -20°C, about -10°C, about 0°C, or higher temperatures).

The enclosure also includes a plate contact mechanisms (235) that includes electrical contact probes mounted onto a plate contact elevator for raising the probes to contact electrical contacts on the bottom of a test plate well. The contact probes are used to apply the electrical potentials to electrodes in a test well induce ECL in the well. The plate contact mechanism and the imaging apparatus are in alignment such that the electrical contact is made to the well that is directly under, and in the imaging field of, the imaging apparatus.

The plate translation stage comprises a plate holder for supporting the plate which has an opening under the plate to allow plate elevators positioned below the plate holder to access and lift the plate and to allow the plate contact mechanism to contact the bottom of the plate. Furthermore, the plate translation stage is configured to position plates, e.g., to position one or more wells of a test plate, below the detection aperture and to position the plates above the plate elevators. The plate translation stage is also configured to position the plate, e.g., one or more wells of a test plate, beneath one or more components of the well wash subassembly, e.g., the imaging apparatus, the plate piercing probe, the well-wash head and the wash station.

In one embodiment, the plate translation stage can accommodate more than one plate at a time, e.g., a multi-plate translation stage. In a specific embodiment, the translation stage is a dual plate translation stage. A multi-plate translation stage enables the apparatus to seamlessly transition between the last stage of an assay on one plate on the translation stage to the beginning of another assay on the next plate on the stage. As described in more detail below, if each plate on the translation stage is a 96 well multi-well assay plate, the instrument will seamlessly transition from beginning analysis of the 96^{th} sample on the first plate to beginning analysis of the first sample on the second plate (i.e., the 97^{th} sample present on the translation stage), without first requiring that the analysis of the 96^{th} is completed. The apparatus tracks the use of each well in each plate and when sample has been dispensed into the last well of a given plate, it transitions sample dispensing from that plate to the next without an interruption in plate processing. After analysis of all of the wells in a first plate is complete, analysis of samples can continue on the second plate on the stage. During this time the first plate can be exchanged with a fresh third plate to enable uninterrupted processing of any arbitrary number of samples.

In one embodiment, the plate translation stage may be used to achieve rapid one or two axis oscillation of plate holder and, thereby, to shake and mix the contents of a plate on the plate holder. The shaking profiles can range from continuous single-axis shaking to duty-cycled orbital shaking. One example includes shaking with the axes at two different frequencies. The apparatus may also provide for sonication to enhance mixing during sample incubation, for example, as described in the U.S. Patent 6,413,783.

The light-tight enclosure may include a light source (e.g., an LED) located underneath the imaging aperture and below the elevation of plate translation stage. In one embodiment, this light source is located on the plate contact mechanism. This arrangement allows for the optional use of fiducial holes or windows in plates to be used to correct for errors in plate alignment. Light from the light source is passed through the fiducials and imaged on the imaging apparatus so as to determine the correct for the alignment of the plate. Advantageously, plates formed from plate bottoms mated to a plate top (e.g., plates with screen printed plate bottoms mated to injection-molded plate tops as described in copending U.S. Applications 2004/0022677 and 2005/0052646) include fiducials patterned (e.g., screen printed) or cut into the plate bottom to correct for misalignment of the plate bottom relative to the plate top. In one specific embodiment, the plate top on such a plate includes holes (e.g., in the outside frame of the plate top) aligned with fiducials on the plate bottom to allow imaging of the fiducials. Accordingly, the imaging of light generated under a plate may be used to communicate the exact position of the plate to the image processing software and also to provide for a camera focus check. The plate may then be realigned using a two-axis positioning apparatus. Thus, the apparatus may process assay test plates via a plate positioning method comprising: (1) providing a plate having light-path openings; (2) illuminating plate from the bottom; (3) detecting light coming through light-path openings; and (4) optionally, realigning the plate.

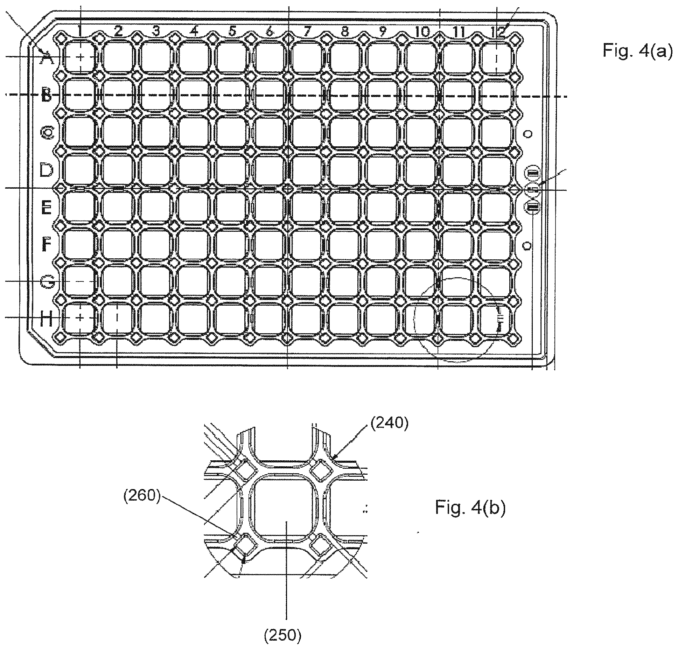

The apparatus of the present invention uses multi-well assay test plates. In one embodiment, the plates are sealed and include desiccant to provide for long term stability outside of their packaging. As shown in Fig. 4(a), an assay plate may include assay wells that are connected to dedicated desiccant spaces located in the regions between the assay wells. Fig. 4(b) provides an expanded view of one well of this assay plate. Each of the desiccant spaces (240) are connected to the surrounding wells (250) via an air passage, e.g., a notch (260). The wells are then sealed with a plate seal (which may be a metalized or foil seal) that is adhered to the plate top (e.g., by an adhesive or through the use of thermal or sonic bonding). The air passages connect the desiccant spaces to the corresponding wells even after the plate top is sealed. For use in electrochemiluminescence measurements, the test plates may have integrated electrodes in the wells. In one such example, plates are formed by mating an injection molded plate top to a plate bottom having screen printed carbon ink electrodes a patterned dielectric layer positioned over the working electrode on the bottom of each well that defines a plurality of "spots" or exposed areas on the working electrode. Reagents for one or more target analytes of interest are immobilized on the different spots within each well of the test plate to allow for measurements in an array format. Suitable assay plates are described in U.S. Patent Application Serial No. 11/642,970, the disclosure of which is incorporated herein by reference.

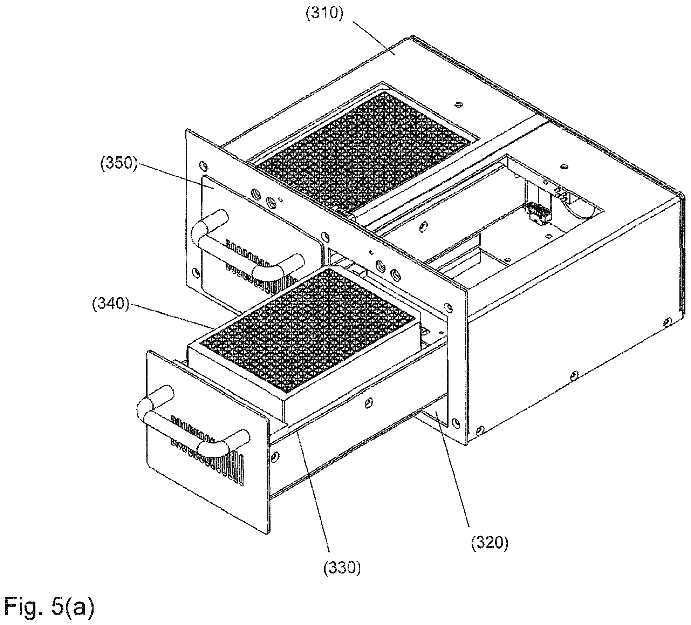

Reagents used in an assay conducted by the apparatus may be stored in an auxiliary multi-well plate, referred to herein as an auxiliary plate, stored and accessed by the apparatus in the auxiliary plate subassembly (Fig. 5(a)). While test plates are capable of storing dry reagents required to perform multiplexed assays, these reagents may alternatively be stored in the auxiliary plates or the auxiliary plates may include additional reagents required for an assay or a step of an assay. The reagents provided in the auxiliary plate may be in liquid and/or dry form. In one embodiment, the reagents are dried. In addition, the auxiliary plates also provide wells (referred to herein as auxiliary wells) for sample dilution and/or sample pre-treatment. Still further, the auxiliary plates may include quality control reagents and/or calibration standards. The wells of the auxiliary plate may be sealed (e.g., with a plate seal) to keep reagents confined to the wells and/or to protect the wells and their contents from the environment.

The auxiliary plate subassembly includes a housing (310) within which is one or more individual compartments (320) with a plate support (330) onto which an auxiliary plate (340) may be placed and an auxiliary plate introduction aperture (350) through which plates may be inserted or removed from the support (manually or mechanically). In one embodiment, the auxiliary subassembly includes two or more separate compartments, each with a plate support and introduction aperture. Each compartment of the auxiliary plate subassembly is individually temperature and humidity controlled via individual heaters/coolers (e.g., a thermoelectric heater/cooler) and/or a desiccant chamber.

The auxiliary plate used in the auxiliary plate subassembly is configured as a multi-well assay plate. The configuration of an auxiliary plate, i.e., the number of auxiliary wells in the plate, is dependent on the assay(s) to be conducted using a given test plate. For example, if a 96 well assay test plate is to be used in an assay, it may be advantageous to use a 384-well auxiliary plate in order to provide multiple auxiliary wells for holding multiple different reagents and/or carrying out multiple reagent/sample processing steps for an assay that will be conducted in a corresponding test well. Accordingly, a test well in an assay test plate may be associated with a "set" of wells in an auxiliary plate which may include a plurality of auxiliary wells that are used to conduct the assay in the test well. In one embodiment, the number of auxiliary wells in an auxiliary plate is a multiple of the number of wells in the test plate, e.g., one, two, or four times the number of test wells. Still further, the identity and arrangement of reagents placed in the wells of the auxiliary plate is determined by the assays to be conducted in each well of the corresponding assay test plate and in each step of that assay. In certain embodiments, the set of auxiliary wells includes a dilution well, i.e., an empty well that is used for a sample dilution step in an assay. The set may further comprise pre-treated beads, which may be magnetic and/or may comprise a coating selected from the group consisting of streptavidin, biotin, and avidin, and one or more reagents in the set of auxiliary wells comprise a binding partner of the coating. If the auxiliary well includes magnetic beads, the auxiliary plate subassembly further includes a magnet (permanent or electromagnetic) that can be used to attract and retain the beads. The magnet may be movable relative to the auxiliary plate or it may be positioned under a well of the auxiliary plate. For example, the magnet may hold beads that have captured analyte in a well of the auxiliary plate while the pipettor performs a wash step to remove unbound material. Alternatively, if the beads are used to remove interferent, the beads may be held in the well while the solution in the auxiliary well is aspirated. In addition, pre-treated beads may also be included in one or more wells of the assay test plate. If a test well includes magnetic beads, the light tight enclosure further includes a magnet that can be used to attract and retain the beads. In one embodiment, the magnet is affixed to or associated with the plate translation stage. The magnet may be used to hold beads in a test well which the apparatus performs one or more additional assay operations, e.g., a wash step and/or ECL generation/detection. Reference is made to U.S. Patent Application Ser. No. 61/212,377, which describes various assay methods that involve the use of magnetic particles or beads to improve assay performance.

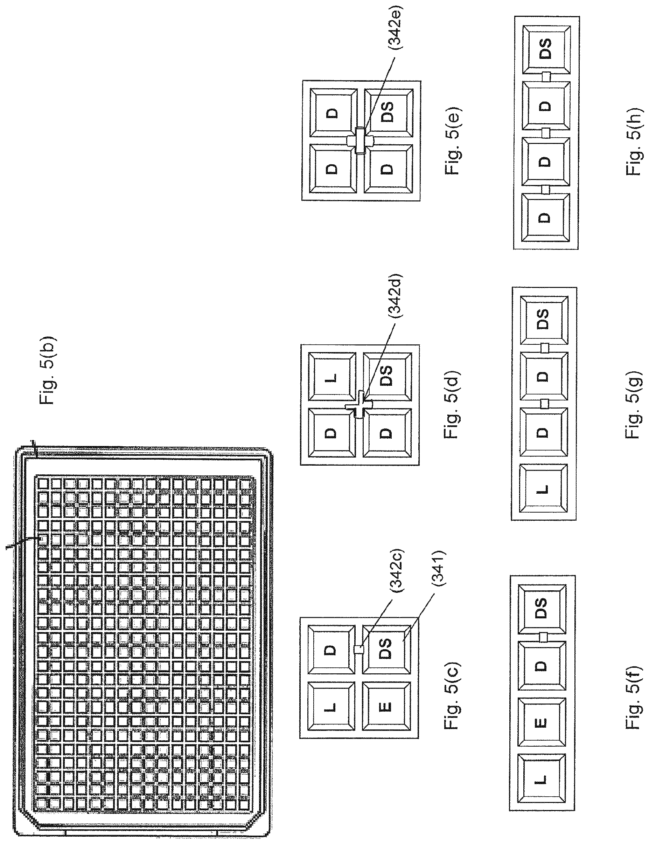

When an auxiliary plate includes auxiliary wells with dry reagents, it may be beneficial to seal the plate with a plate seal and/or to include additional wells containing desiccant to maintain the dry reagents in a dry state when the plate is out of its packaging. The seal, preferably, has a low water vapor permeability to prevent evaporation of liquid reagents and/or to keep dry reagents dry and may comprise a metalized substrate or a metal foil. As described above for assay plates, individual auxiliary wells within a set of auxiliary wells may be linked to a desiccant well in that set by air passages (such as notches in the well walls) such that desiccant wells can maintain the linked wells in a dry state when the plate is sealed. Figures 5(c) through 5(h) show different configurations of sets of four auxiliary wells (341) having one desiccant well. In these configurations, notches in the well walls - e.g., notches 342c, 342d or 342e - are used to connect one well (Figures 5(c) and 5(f)), two wells (Figures 5(d) and 5(g)) or three wells (Figures 5(e) and 5(h)) to the desiccant well in the set. The wells in each set could be arranged in a variety of ways (where the wells in a set may be adjacent to each other or not) such as a square block of wells (as in Figures 5(c), 5(d) or 5(e)) or in a linear arrangement (as in Figures 5(f), 5(g) or 5(h)), as long as the appropriate air paths can be provided.

A set of auxiliary wells may include liquid and/or dry reagents. In certain embodiments, each assay well may be associated with a set of auxiliary wells that may include a combination of liquid or dry reagents. For example, certain reagents, e.g., detection antibodies, may be provided in a first well of a set of auxiliary wells in dried form, and another well of that set of auxiliary wells includes an assay diluent in liquid form. If a mixture of liquid and dry reagent is provided in a set of auxiliary wells, a desiccant well may be provided, but the air passage connecting the desiccant well to the other auxiliary wells is designed to isolate (i.e., not connect to) wells containing liquid or to only connect to those auxiliary wells including dry reagents. For example, a set of auxiliary wells including liquid and dry reagents may comprise four auxiliary wells including: (1) dried detection antibodies; (2) desiccant; (3) liquid assay diluent; and (4) a dilution (empty) well, and an air passage will be provided in the set of auxiliary wells that connects wells (1) and (2), but does not connect to wells (3) and (4). In an alternate embodiment, liquid reagents for use in one or more assays involving one or more sets of auxiliary wells in the auxiliary plate may be provided in a contiguous region of the auxiliary plate, e.g., in a single contiguous row of auxiliary wells of the auxiliary plate. Various configurations of a set of auxiliary wells are provided in Figs. 5(c)-5(h). Figs. 5(c) and 5(f) illustrate embodiments of a set of auxiliary wells which includes a first well with dried detection antibodies (D), a second well with desiccant (DS), a third well with liquid reagent (L), and an empty well (E) for dilution (alternatively, "E" wells may contain dry assay diluent). An air passage is provided between the first well and the second well so that the desiccant controls the humidity in the first well, but the second well is not connected to the third well or the empty well. Alternatively, Figs. 5(d) and 5(g) illustrates embodiments of a set of auxiliary wells which includes a first and second well each with dried detection antibodies (D), a third well with desiccant (DS) and a fourth well with liquid reagent (L). The third well is connected to each of the first and second wells but not to the fourth well. In further embodiments (shown in Fig. 5(e) and Fig. 5(h)), three of the four wells comprise dried reagents and the fourth well includes desiccant and all of the wells in the set are connected to the desiccant well via an air passage. For each assay conducted in a single well of the test plate, a set of auxiliary wells are suitably configured. Exemplary configurations of auxiliary wells for various assays that may be conducted in a single well of an assay test plate are described in Table 1, below.

**Table 1**

| **Test Panel Example** | **Sample Prep** | **Assay Plate Incubation Steps** | **Assay Plate** | **Auxiliary Plate** |
|---|---|---|---|---|
| One step sandwich binding assay | - | 1-Step | 1-Well panel: Specific capture reagents | Two well sets with |
| | | | | 1. Detection reagents/assay buffer |
| | | | | 2. Desiccant |
| One step sandwich binding assay with sample prep step | Pretreatment | 1-Step | 1-Well panel: Specific capture reagents | Three well sets with |
| | | | | 1. Sample prep reagent |
| | | | | 2. Detection reagents/assay buffer (optionally + quenching reagent) |
| | | | | 3. Desiccant |
| One step sandwich binding assay with sample dilution step | Dilution | 1-Step | 1-Well panel: Specific capture reagents | Three well sets with |
| | | | | 1. Dilution well |
| | | | | 2. Detection reagents/assay buffer (optionally + quenching reagent) |
| | | | | 3. Desiccant |
| One step sandwich binding assay with two well assay panel | - | 1-Step | 2-Well panel: | Two well sets with |
| | | | 1. Panel 1 capture reagents | 1. Detection reagents/assay buffer |
| | | | 2. Panel 2 capture reagents | 2. Desiccant Two types of sets on each plate for the two different assay panels |
| Customizable assay panels | - | 1 or 2-Step | 1-Well panel: Standard set of capture reagents | Three well sets with |
| | | | | 1. Specific capture reagents |
| | | | | 2. Detection reagents |
| | | | | 3. Desiccant |
| Two step sandwich binding assay | - | 2-Step | 1-Well panel: Standard set of capture reagents | Three well sets with |
| | | | | 1. Assay buffer components |
| | | | | 2. Detection reagents/assay buffer |
| | | | | 3. Desiccant |
| Serology panel | Dilution | 2-Step | 1-Well panel: Set of antigens | Four well sets with |
| | | | | 1. Dilution well |
| | | | | 2. Dilution well (optional, for 2^{nd} dilution) |
| | | | | 3. Detection reagents/assay buffer |
| | | | | 4. Desiccant |
| Immunoassay for influenza with two well panel (typing and subtyping) and acidic pretreatment prior to subtyping analysis | Acid treatment | 1-Step | 2-Well panel: | A two well typing set with |
| | | | 1. Flu detection/typing panel (array of | 1. Detection reagents/assay buffer (including surfactant for lysing virus) |
| | | | capture antibodies for Flu A nucleoprotein | 2. Desiccant A three well subtyping set with |
| | | | and Flu B nucleoprotein) | 1. Acidification buffer (including surfactant) |
| | | | 2. Flu A subtyping panel (array of capture antibodies for hemagglutinin subtypes (HI, H2, H3, etc.) | 2. Detection reagents/assay buffer (including neutralization buffer) |
| | | | | 2. Desiccant |

Table 1 and the description below provides many examples of consumables, kits and methods that are used with sandwich binding assays employing capture and detection binding reagents that bind targets of interest. One of average skill in the art will be able to select suitable binding reagents which could include antibodies or nucleic acids. The kit format is also readily modifiable to other binding assay formats. For example, to conduct a competitive assay, the capture reagent is selected to compete with a target of interest for binding to the detection reagent (or, alternatively, the detection reagent is selected to compete with the target for the capture reagent).

Therefore, a basic one step sandwich immunoassay for a target analyte (i.e., an assay in which the assay target is bound to both the capture and detection reagents in a single incubation step) may be conducted in a single well of an assay test plate. In one such embodiment, the test plate will include capture antibodies and the corresponding set of auxiliary wells in the auxiliary plate will include a first auxiliary well with detection antibodies and components of an assay buffer (e.g., the buffers, salts, blocking agents, detergents, etc. that provide the optimal environment for binding the capture/detection reagents to the targets). The other well in the set will include desiccant.

Alternatively, the consumables may be configured to carry out two different assays or panels of assay on a sample in two different wells of an assay plate, in which case, the auxiliary plate may have two types of sets of auxiliary wells for the two panels. The consumables may also be configured to carry out two-step assay in which the target is bound to a capture reagent in a first step and the resulting complexes are bound to a detection reagent in a second step. The consumables may also be configured to include sample processing wells in the auxiliary plate to allow for a sample dilution or preparation step.

In one embodiment, the test plate is configured in a "generic" customizable format and the target(s) of an assay are determined based on the reagents included in the auxiliary plate. For example, a well of the test plate may include a binding partner for a ligand, or for a customizable multiplexed assay, a plurality of binding partners for a plurality of different ligands. The binding partners, e.g., may be immobilized to one or more spots within a test well to form an array. Capture reagents for a specific assay or multiplexed assay panel are provided in the auxiliary plate and are designed such that different capture reagents comprise different ligands. This approach enables in situ formation of arrays directed through the binding of the ligands in the capture reagents to their binding partners. Analogously, the same approach can be used to direct specific capture reagents to assay particles coated with different binding partners (for particle-based assays), where the particles could be provided in the assay test well, in the auxiliary plate or as a separate reagent. Suitable binding partner - ligand pairs include, but are not limited to, complementary nucleic acid sequences, antibody-antigen pairs, antibody-hapten pairs and other receptor ligand pairs (such as avidin-biotin or streptavidin-biotin). In one embodiment, a capture reagent comprises a material that binds a target of interest (e.g., an antibody) that is chemically linked to the ligand (e.g., by reacting the material with an active ester of the ligand). In one embodiment of an assay using a generic test plate with an array of binding partners, the accompanying auxiliary plate includes a set of wells comprising capture antibodies (comprising the appropriate ligands), detection antibodies, optional dilution wells, and desiccant.

The invention includes methods and consumables and kits for carrying out assays detecting influenza infections (e.g., as described in the last row of Table 1 and in Example 3). In particular, applicants have discovered that the sensitivity of assays for detection of influenza and/or for determining influenza subtype by detection of influenza hemagglutinin proteins can be significantly enhanced by pretreatment of the samples under acidic conditions (pH 4.0 to 5.2 or 4.5 to 5.0) and incubating for a period of time (in one embodiment, between 1 second and 4 8 hours, in another embodiment between 5 seconds and 10 minutes, in another embodiment between 15 seconds and 5 minutes). The acidification can be achieved by combining a sample with a dry or liquid acidification reagent that, when combined with the sample, brings the sample to the correct pH. Suitable acidification reagents include strong acids such as hydrochloric and sulfuric acid. Advantageously, the acidification reagent is a buffered solution at or near the desired pH that includes a buffering agent with buffering capacity in the appropriate pH range (e.g., appropriate buffering agents include, but are not limited to, ones based on carboxylic acids such as acetic acid and lactic acid and, especially, polycarboxylic acids such as citric and glutaric acid and also include quaternary ammonium buffers such as MES). In one embodiment, the concentration is between 50-500 mM or between 100 and 200 mM or around 117 mM and the pH of the buffer is between 4.0 to 5.2 or 4.5 to 5.0, where the concentration/pH are defined as i) the concentration/pH of a liquid acidification reagent, ii) the concentration/pH of a liquid acidification reagent from which a dry reagent is prepared (e.g., by lyophilization) or iii) the final concentration/pH achieved after combining a sample with a liquid or dry acidification reagent. The reagent may also include a surfactant (e.g., a non-ionic surfactant such as Tween 20, Thesit, Triton X-100 or an ionic surfactant such as deoxycholic acid or CHAPSO), preferably at a concentration near to or greater than the CMC. In one embodiment, the reagent includes greater than 0.02% Triton X-100 or greater than 0.05% Triton X-100 or about 0.1% Triton X-100. In one embodiment, the pH of the sample is at least partially neutralized prior to or during analysis of the sample by immunoassay. The method may therefore include treatment of the acidified sample with a neutralization reagent that brings the pH to pH 6.0 or greater, pH 6.5 or greater or pH 7.0 or greater. The neutralization reagent may be a strong base such as sodium or potassium hydroxide or a buffering agent with buffering capacity in the appropriate pH range (e.g., HEPES, phosphate, Tris, etc.). In one embodiment, the concentration is between 50-1000 mM or between 100 and 400 mM or around 253 mM and the pH of the buffer is between 6.0 to 8.5 or 6.5 to 8.0, where the concentration/pH are defined as i) the concentration/pH of a liquid acidification reagent, ii) the concentration/pH of a liquid acidification reagent from which a dry reagent is prepared (e.g., by lyophilization) or iii) the final concentration/pH achieved after combining a sample with a liquid or dry acidification reagent. The neutralization reagent may be provided in an assay test well or as a separate reagent and may also include other assay components such as blocking agents, surfactants, salts, detection antibodies, etc. In one embodiment, the acidification and neutralization reagents are provided as dry reagents in separate wells of an auxiliary plate. Alternatively, the one or both reagents (e.g., the neutralization reagent) is provided as a liquid reagent in a well of the auxiliary plate or in a separate reagent container.

The assay formats described in Table 1 are non-limiting examples, and the skilled artisan will appreciate the numerous possible permutations of test plate and auxiliary plate configurations.

Each auxiliary plate includes an identifier that is used to identify the plate or reagents in the plate. The identifier, as described below, may be, e.g., a bar code, an EEPROM, or an RFID. In one embodiment, the identifier is an EEPROM and the auxiliary plate subassembly includes an identifier controller to read and process the data stored to the EEPROM. In another embodiment, the user inputs auxiliary plate specific information (either manually or by uploading such information from an external user computer and/or network) into the user interface and the instrument associates that information with each auxiliary plate. As described below, the data stored to the identifier may include assay consumable information that identifies additional assay consumables, e.g., test plates, liquid reagents or diluents, which should be used for a given assay using that auxiliary plate. Accordingly, when the identifier controller processes the information stored to the identifier on the auxiliary plate, the apparatus will inventory the consumables present in the apparatus and prompt the user if one or more additional consumables identified on the auxiliary plate identifier is not present in the apparatus.

Therefore, the invention provides an auxiliary plate comprising a plurality of auxiliary wells each comprising dry assay reagents for use in an assay with a corresponding assay test plate. In one embodiment, the auxiliary plate comprises an identifier comprising assay information used to identify an element selected from the group consisting of (i) the auxiliary plate, (ii) one or more auxiliary wells within the auxiliary plate, (iii) a reagent and/or sample that has been or will be used with the auxiliary plate, (iv) the test plate, (v) one or more wells within the test plate, (vi) a reagent and/or sample that has been or will be used with the test plate, and (vii) combinations thereof. The assay consumable information that may be stored, erased from and/or written to the EEPROM on the auxiliary plate during the conduct of an assay or a series of assays includes but is not limited to any information used to uniquely identify a particular assay or assay step, assay consumable, consumable domain(s), biological reagent or sample or to distinguish a particular assay, assay step, assay consumable, consumable domain(s), biological reagent or sample from other assay consumables, consumable domains, biological reagents or samples. In one embodiment, the assay consumable information that is stored, erased from and/or written to the EEPROM on the auxiliary plate includes information concerning individual well usage, i.e., when and how an auxiliary well is used in a particular assay, to allow the user to track usage of the auxiliary plate. In addition, assay information may include consumable information, sample information, chain of custody information, consumable/test well information, auxiliary well or set information, assay process information, consumable security information, and combinations thereof. Each type of assay information is described in more detail below.

The invention also provides a kit including a multi-well assay test plate and an auxiliary plate, e.g., the test plates and corresponding auxiliary plates as described elsewhere in this application. The test plate includes a plurality of assay wells and the auxiliary plate includes a plurality of auxiliary wells wherein the auxiliary wells comprise assay reagents for use in an assay with the test plate. In one embodiment, the assay reagents are dry. The number of auxiliary wells may be a multiple of the number of test wells, e.g., one, two, or four times the number of test wells. Each plate of the kit, i.e., the test plate, the auxiliary plate, or both, includes an identifier, e.g., a bar code, and EEPROM or an RFID, which includes assay information for that consumable. For example, the assay information identifies the auxiliary plate (e.g., by lot or unique auxiliary plate identification number), the test plate (e.g., by lot or unique test plate identification number), a set of auxiliary wells within the auxiliary plate that should be used with a given well of a test plate, the identity of a reagent and/or sample that has been or will be used with the auxiliary plate, a set of wells in the auxiliary plate, a test plate, or one or more wells of the test plate.

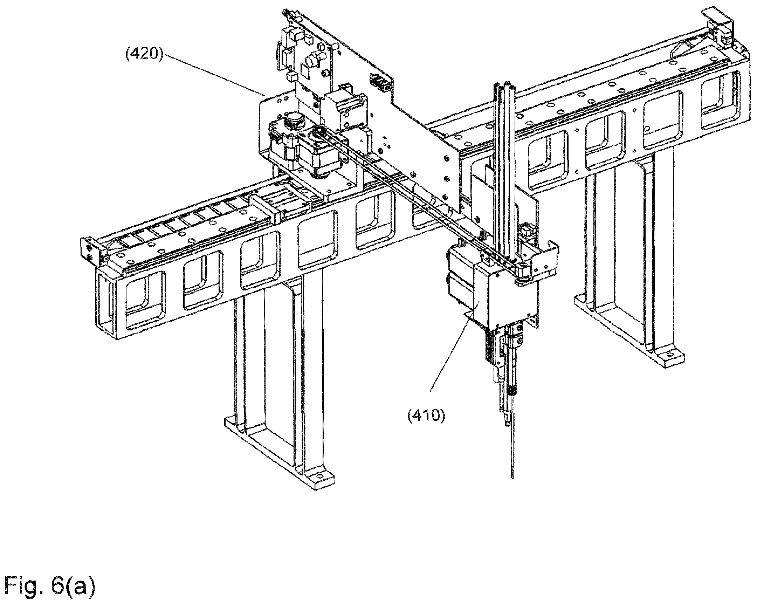

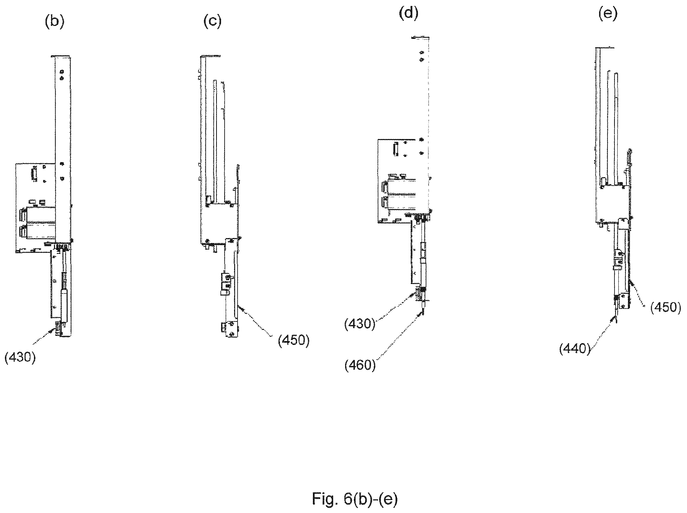

The pipettor subassembly (Fig. 6(a)) is used to transfer samples and reagents between sample rack subassembly, the auxiliary plate subassembly and test plates in the light-tight enclosure. The pipettor subassembly (Fig. 6(a)) includes a probe assembly (410) affixed to a pipettor translation gantry (420) that provides X and Y motion. The probe assembly includes (a) at least one pipetting probe, (b) an auxiliary plate well piercing probe and (c) an ultrasonic sensor. The probe assembly provides for independent Z (vertical) motion of the different probes so as to allow them to access sample tubes, test plates and/or auxiliary plates (as required). The pipettor subassembly also includes the appropriate pumps and valves for controlling the pipettors (not shown).

In one embodiment, the pipettor subassembly also includes a pipetting tip sensor to determine if there is a pipetting tip on the pipetting probe. The pipetting tip sensor may comprise a reflective sensor positioned in the subassembly to detect pipetting tip pick-up and ejection. Alternatively or additionally, the pipetting tip sensor may include a light transmission sensor with source and detector on opposite sides of the pipetting probe. In a specific embodiment, the pipetting tip sensor is a reflective sensor affixed to the pipetting probe, e.g., adjacent to the pipetting probe tip and optionally offset from, e.g., just below the pipetting probe tip. The pipetting tip sensor components may be mounted on the probe assembly or elsewhere in the system, as long as the components are accessible to the pipetting probe. The reflective sensor may include an infra-red LED and a phototransistor, wherein the LED and phototransistor are positioned side-by-side such that the presence of an object in front of the sensor is detected by the light reflected by the object into the sensor phototransistor. The presence or absence of the pipette tip is detected by checking the electrical output of the phototransistor circuit. If a pipette tip is present, sufficient light is reflected onto the phototransistor to cause it to turn on, whereas the absence of a pipette tip causes the phototransistor to turn off. In one embodiment, the reflective sensor is positioned to enable continuous monitoring of the presence or absence of the pipette tip. The operating software for the assay system can periodically check the state of the sensor at any time. In another embodiment, a light source, preferably collimated, is used to simulate an optical trip-wire. A light detector is positioned in the subassembly to detect the interruption of the light path. The light path is selected to intersect the path taken by the pipetting probe immediately after it has picked up a new pipetting tip or disposed of a used pipetting tip. The output of the detector is used to detect the presence or absence of a disposable tip. Still further, an optical interrupter switch or a reflective sensor may be mounted to a fixed position in the subassembly.

A specific embodiment of a pipetting tip sensor is shown in Figs. 6(b)-(e). Figs 6(b)-(c) show the side and front views, respectively, of the pipetting subassembly including a pipetting tip sensor comprising a reflective sensor (430), a sensor PCB (440), and a bracket (450). Figs. 6(b)-(c) show the subassembly without a pipetting tip. Figs. 6(d)-(e) are side and front views, respectively, of the pipetting subassembly including a disposable tip (460) attached to the pipetting probe and in proximity to the sensor (430).

A pump is used to drive fluids through the pipetting apparatus. One skilled in the art will be able to select appropriate pumps for use in the apparatus including, but not limited to diaphragm pumps, peristaltic pumps, and syringe (or piston) pumps. The pump also includes a multi-port valve to allow the pump to push and pull fluids from different fluidic lines. Alternatively, multiple pumps can be used to independently control fluidics in different fluidic lines.

The piercing probe is used to pierce and displace seals on wells of the auxiliary plate. The piercing probe is, preferably, pointed to facilitate piercing. Optionally, the probe tip is pyramidal in shape (e.g., a square pyramid) to provide cutting surfaces that provide for reproducible cutting of the seal into sections as the probe is inserted. In one embodiment, the probe shaft is slightly undersized relative to the auxiliary wells and/or has roughly the same shape as the wells so that the cut sections of the seal are folded against the internal walls of the well.

In one embodiment, the pipetting probe uses disposable pipetting tips that are stored in a pipetting tip storage/disposal compartment. The arm/track of the pipettor subassembly allows for access of the probe to the tip storage/disposal compartment for tip loading on the pipetting probe and tip removal after use. The use of disposable pipetting tips reduces cross-contamination and it eliminates sample carry-over. Alternatively, a fixed tip pipettor may be used. In one embodiment, the probe assembly includes both fixed tip and disposable tip pipettors. In addition to transferring reagents and samples from one tube/well to another, the fluidic lines leasing to the pipetting probes may also be connected to working fluids or diluents so that the probes can be used to deliver these fluids/diluents to tubes or wells. Optionally, the pipetting probes may include fluid sensing capability, e.g., using capacitive sensors to detect when the probes contact fluid in a tube or well.

The ultrasonic sensor is used to measure the height of a surface or liquid under the sensor. The use of an ultrasonic sensor improves pipetting accuracy. Sample delivery may be configured by measuring fluid height within a well using the ultrasonic sensor. Still further, the ultrasonic sensor may also monitor the positioning of the pipetting probe within a well of a multi-well plate by adjusting the probe's position upon contact with the walls of the well. The pipetting probe may also include a three-axis force sensor in-line with the pipetting axis to improve pipetting accuracy. The force sensor may also be used for self-alignment/training of the pipetting probe. In addition, the ultrasonic sensor can be used to (a) detect whether the foil seal of a well has been pierced; (b) count disposable pipetting tips in the tip compartment; (c) confirm that the tip boxes and/or auxiliary plates are loaded into the apparatus correctly; (d) detect the presence of capped sample tubes; and (e) detect or confirm the presence of sufficient sample in sample tube and/or within a well of a plate. Alternatively, one or more of these functions may be performed by an optional optical sensor.

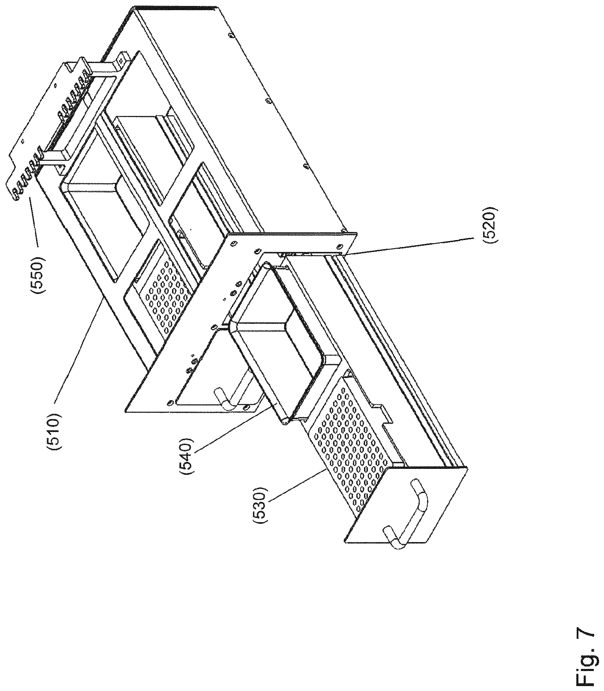

Disposable pipetting tips are stored and disposed of in the tip compartment, shown in Fig. 7. The tip compartment includes a housing (510) for one or more individual drawers (520) that can accommodate a standard disposable tip box (530) (available from Axygen, Qiagen or Rainin) and a removable waste container (540) for used pipetting tips. A multi-position tip removal bracket 550 is also included. The bracket has U-shaped slots that are sized to be wider than the pipettor shaft but narrower than the widest region of the disposable tips. To remove tips, the pipettor probe is translated horizontally to locate the shaft in the slot and then translated vertically until the pipette tip is pulled off by the bracket. During operation, the specific slot that is used is chosen using a set pattern or a random pattern such that the used pipette tips are distributed evenly along the width of the waste container. The dimensions of the tips vary according to the dimensions of the pipetting probe, the volume of the sample/reagents dispensed and/or the dimensions of the plates within which the tip is placed. In one embodiment, the tip volume ranges from approximately 100 µL to 500 µL. In another embodiment, the tip volume ranges from about 200 µL to 300 µL. In a particular embodiment, the tip volume is about 250 µL.

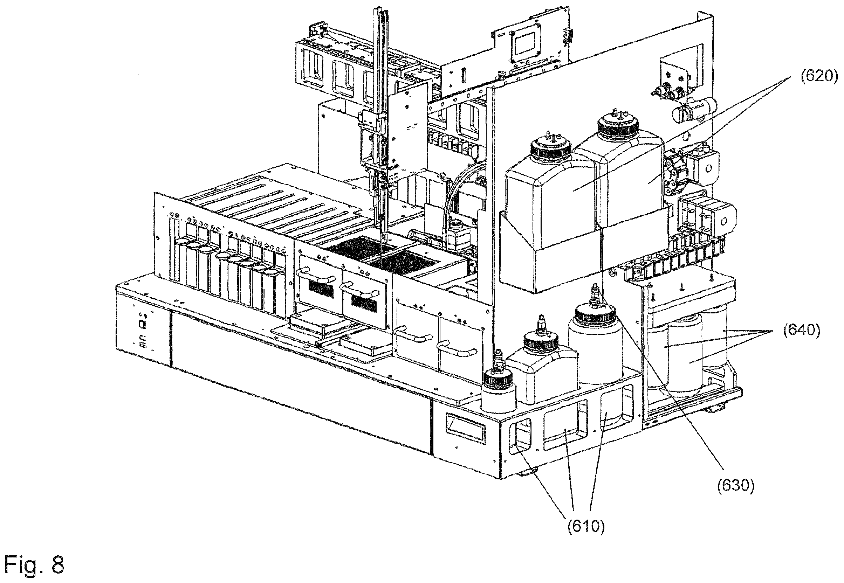

The liquid reagent subassembly (Fig. 8) includes a plurality of liquid reagent (610) and waste (620) compartments and for use in one or more steps of an assay conducted in the apparatus. A reagent/waste compartment comprises a compartment body that encloses an internal volume and a reagent or waste port (630) for delivering reagent or receiving waste. The volume of the compartments in the subassembly are adjustable such that the relative proportion of the volume of the compartment body occupied by reagent and waste can be adjusted, e.g., as reagent is consumed in assays and returned to a compartment as waste. The total internal volume of the compartment body may be less than about 2, less than about 1.75, less than about 1.5, or less than about 1.25 times the volume of liquid stored in the body, e.g., the volume of reagent originally provided in the compartment, thus minimizing the space required for waste and reagent storage, and allowing for convenient one-step reagent replenishment and waste removal. In certain embodiments, the apparatus has a reagent compartment slot configured to receive the compartment, and provide fluidic connection to the waste and reagent ports, optionally via "push-to-connect" or "quick connect" fittings.

Optionally, the reagent and/or waste compartments are removable. In one embodiment, the reagent and/or waste compartments are removable and the apparatus further includes a sensor, e.g., an optical sensor, to monitor the fluid level(s) in the reagent and/or waste compartments. Alternatively, the liquid reagent subassembly may include electronic scales to monitor the weight of fluid in the reagent and waste reservoirs for real-time tracking of reagent use and availability. Once the reagent and/or waste compartments reach a certain minimal or maximal capacity, as detected by the sensor or scale, the apparatus alerts the user to remove the reagent or waste compartment to replenish and/or empty the contents. In one embodiment, the motor of the pipetting probe is in communication with the sensor or scale and when the reagent and/or waste compartments reach the minimal or maximal capacity, the pipetting probe motor is disabled by the apparatus, e.g., the probe sensor relays information regarding the capacity of the compartment to the instrument software, which then halts further pipetting action.

The reagent and waste compartments may be provided by collapsible bags located in the subassembly body. One of the reagent and waste compartments may be provided by a collapsible bag and the other may be provided by the compartment body itself (i.e., the volume in the compartment body excluding the volume defined by any collapsible bags in the compartment body). In addition to the first reagent and waste compartments, the reagent cartridge may further comprise one or more additional collapsible reagent and/or waste compartments connected to one or more additional reagent and/or waste ports. Alternatively, one or the other of the reagent and waste compartments may be constructed from blow-molded plastic.

In one embodiment, the liquid reagent subassembly also includes a reagent reservoir (640) that is used during the conduct of an assay in the apparatus. In one specific embodiment, each reagent compartment is connected via a fluidic line to a reagent reservoir that houses a volume of reagent used during the assay. Fluidic lines to the pipettor subassembly and the well-wash subassembly lead directly from the reagent reservoir. In practice, reagent is stored in a reagent compartment and a predetermined volume of reagent is dispensed from the reagent compartment to the reagent reservoir. The apparatus draws fluids for use in an assay from the reagent reservoir. The reagent compartment and reagent reservoir are each connected to an independent fluid sensor. The fluid sensor in the reservoir monitors the internal volume within the reservoir and if the internal volume decreases below a predetermined level, reagent is dispensed from the reagent compartment to the reservoir. Likewise, if the internal volume of the reagent compartment decreases below a predetermined level, the fluid sensor signals to the operator to replace or refill the reagent container. The dual reagent compartment/reservoir assembly enables the apparatus to continually supply fluid to an assay as the assay is conducted by the apparatus as fluid is replaced in the reagent compartment without interrupting the assay processing by the instrument.

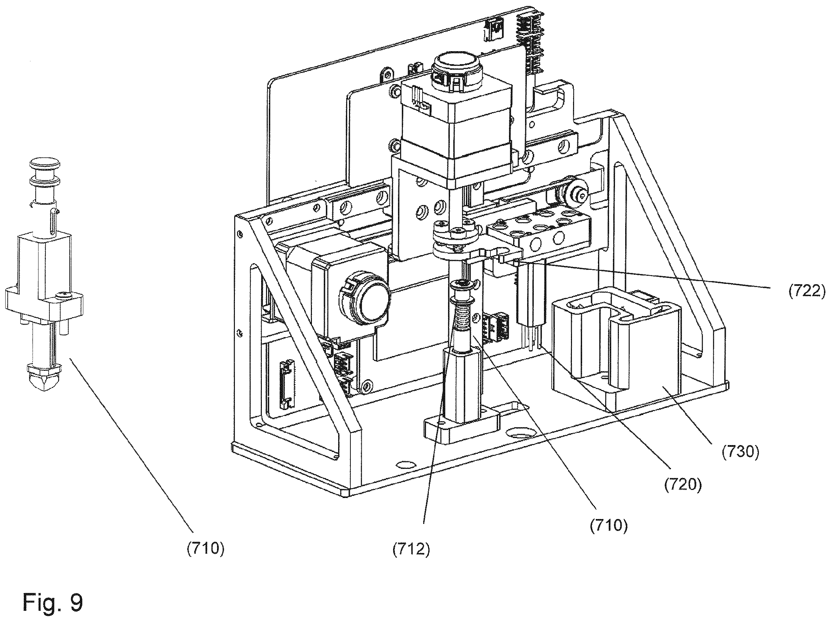

The well wash subassembly, shown in Fig. 9, includes a piercing probe (710), a well-wash head (720), and a wash station (730). The well wash subassembly also includes fluidic connections to the liquid reagent subassembly. The well wash subassembly is used to pierce the foil sealing the wells of the test plate, deliver fluids to the test plate, wash the pipetting probe of the pipetting subassembly, and dispose of waste from the pipettor.

Like the piercing probe in the pipettor subassembly, the piercing probe of the well wash subassembly is used to pierce and displace seals on wells of the test plate. In one embodiment, the well wash subassembly also includes a seal removal tool to remove a seal from a well of a test plate. Removing a seal may include piercing the seal on a well of a test plate and, optionally, cutting the seal into sections (e.g., with using cutting edges on a piercing tip) and folding the sections against the internal walls of the well. In one embodiment, the seal removal tool is a piercing probe that comprises i) a piercing section with external surfaces that taper to a vertex so as to form a piercing tip at one end of a piercing direction (the axis of translation during a piercing operation) and ii) a seal displacement section, arranged adjacent to the piercing section along the piercing direction. In certain specific embodiments, the seal displacement section has a cross-sectional shape, perpendicular to the piercing direction, which is selected to substantially conform to the shape of the openings of the wells on which the probe will operate. The probe may be slightly undersized relative to the well opening so as to allow the probe to slide into the well opening, and press or fold the pierced seal against the well walls. Such an approach may be used to remove the seal as a barrier to detecting assay signals in the well using detectors (for example, light detectors and/or light imaging apparatus') situated above the well. The appropriate clearance may be selected based on the thickness of a specific film and/or may be selected to be less than about 0.1 inches, less than about 0.2 inches, or less than about 0.3 inches.

In one example of a piercing probe, the cross-sectional shape of the seal displacement section is a circle. In another example, it is a square or a square with rounded corners. The piercing section may be conical in shape. Alternatively, it may include exposed cutting edges that, e.g., extend in a radial direction from the tip and can act to cut the seal during piercing and aid in reproducibly folding the seal against the well walls. In one specific example, the tip is pyramidal in shape, the edges of the pyramid providing exposed cutting edges.

In certain embodiments, the piercing probe is spring loaded such that the maximal downward force, along the piercing direction, of the probe on a plate seal is defined by the spring constant of a spring. The probe may also comprise a plate stop section adjacent to the seal displacement section that defines the maximum distance of travel of the piercing probe into the wells. In one specific example, the stop section is a region of the probe with a width that is too large to enter a well and the maximum distance is defined by the distance at which the stop section hits the top of the well.

The well wash head is configured to wash wells by aspirating fluid from wells and replacing it with fresh clean fluid. In one specific embodiment, the wash head is mounted on a translation gantry for translating the pipetting probe in a vertical direction and, optionally, in one or more horizontal directions. Furthermore, the enclosure top has one or more pipetting apertures and the sliding light-tight door has one or more pipetting apertures. The sliding light-tight door has a pipetting position where the pipetting apertures in the enclosure top align with the pipetting apertures in the sliding light-tight door. The pipette translation stage is mounted on the enclosure top and configured such that, when the sliding light-tight door is in the pipetting position, the pipetting probe may be lowered to access wells positioned under the pipetting apertures in the enclosure top.

The wash head comprises one or more vertical tube elements (probes) that include a lower opening through which fluid is dispensed or aspirated. In one embodiment, the lower opening is a blunt tube end. Optionally, the end may be slotted to allow movement of fluid through the opening when the opening is pressed against a flat surface. In certain embodiments, the dispenser comprises two or more tube elements. In one specific example different reagents are dispensed through different tube elements. In another specific example, one tube element is used to dispense reagent and another tube element is used to aspirate waste. Multiple tube elements may be configured in a variety of arrangements, for example, as parallel tubes or concentric tubes.

In one embodiment, the well wash head includes a multi-tube array that comprises one or more dispensing tube elements at the center of the array and a plurality of aspiration tube elements around the periphery of the array. In a specific embodiment, the array includes two dispensing tube elements at the center of the array each comprising an independent fluid channel for buffers and/or diluents used during an assay. The aspiration tube elements surround the dispensing tube elements and are positioned to align with the outer portions of a well bottom of a multi-well test plate. In one specific embodiment, the wells of the test plates are square and the dispensing tube elements of the multi-tube array are configured in a square to align with the inside of the four corners of a well of the test plate. Alternatively, the wells of the test plate are circular and the dispensing tube elements are configured in a circle to align with the inside of four approximately equidistant positions around the inner circumference of a well of the test plate. Preferably, the fluidic lines to each of the aspiration tube elements are linked to independent pumps or vacuum sources (or a single high capacity vacuum source) such that the vacuum on each line is not affected by whether the other lines are pulling vacuum or air. In one embodiment, the dispensing tube elements are surrounded by four aspiration tube elements and the dispensing tube element may be positioned at a different height (e.g., at a greater height) than the aspiration tube elements relative to the well bottom. In practice, a buffer or diluent is dispensed from the dispensing tube element and the aspiration tube elements aspirate fluid from the four corners of the well. This configuration prevents fluid droplets from adhering to the outer positions of the well bottom. In one specific embodiment, two dispensing lines are included: a line for dispensing a wash fluid during wash steps and a line for dispensing an assay read buffer (for example an ECL read buffer) prior to analysis of the well.

The invention includes methods for using the pipetting apparatus for adding or withdrawing fluid from a container, e.g., a well of a multi-well plate. One method involves a continuous wash and comprises (a) lowering the pipetting probe into the container by lowering the translation stage until the aspiration probes are at a first predetermined height above the bottom of the well touches a bottom surface of the container, (b) aspirating fluid from the wells through the aspiration probes on the head; (c) raising the stage until the aspiration probes are at a second higher pre-determined height; (d) continuously dispensing wash buffer from a dispensing probe while aspirating fluid from the aspiration probes to generate a continuous washing action; (e) lowering the translation stage to the first pre-determined height; (f) aspirating fluid from the wells through the aspiration probes and (g) raising the wash head from the well. Another method uses a discrete wash and comprises (a) lowering the pipetting probe into the container by lowering the translation stage until the aspiration probes are at a first pre-determined height above the bottom of the well touches a bottom surface of the container, (b) aspirating fluid from the wells through the aspiration probes on the head; (c) optionally, raising the stage until the aspiration probes are at a second higher pre-determined height; (d) dispensing a pre-determined volume of reagent from a dispensing probe into the well; and (e) raising the wash head from the well. Increasing wash quality can be achieved by combining one or more continuous and/or discrete wash steps, optionally, including a plate shaking step between washes.

In one embodiment of the invention, the wash head translation gantry may also be used to operate the test plate well piercing tool. As shown in Fig. 9, wash head (720) includes a slotted tab (722) that can be translated over so that it engages groove (712) in piercing tool (710). Partial lowering of the wash head can then be used to lower the piercing tool into the light tight enclosure so as to pierce a well of an assay plate.

In addition, the pipetting probe of the well-wash subassembly may be equipped with an ultrasonic, optical and/or force sensor to confirm accurate fluid delivery into a test well.

A method is provided for using the apparatus for conducting measurements in multi-well plates. The plates may be conventional multi-well plates. Measurement techniques that may be used include, but are not limited to, techniques known in the art such as cell culture-based assays, binding assays (including agglutination tests, immunoassays, nucleic acid hybridization assays, etc.), enzymatic assays, colorometric assays, etc. Other suitable techniques will be readily apparent to one of average skill in the art.

Methods for measuring the amount of an analyte also include techniques that measure analytes through the detection of labels which may be attached directly or indirectly (e.g., through the use of labeled binding partners of an analyte) to an analyte. Suitable labels include labels that can be directly visualized (e.g., particles that may be seen visually and labels that generate an measurable signal such as light scattering, optical absorbance, fluorescence, chemiluminescence, electrochemiluminescence, radioactivity, magnetic fields, etc). Labels that may be used also include enzymes or other chemically reactive species that have a chemical activity that leads to a measurable signal such as light scattering, absorbance, fluorescence, etc. The formation of product may be detectable, e.g., due a difference, relative to the substrate, in a measurable property such as absorbance, fluorescence, chemiluminescence, light scattering, etc. Certain (but not all) measurement methods that may be used with solid phase binding methods according to the invention may benefit from or require a wash step to remove unbound components (e.g., labels) from the solid phase.

In one embodiment, a measurement done with the apparatus of the invention may employ electrochemiluminescence-based assay formats, e.g. electrochemiluminescence based immunoassays. The high sensitivity, broad dynamic range and selectivity of ECL are important factors for medical diagnostics. Commercially available ECL instruments have demonstrated exceptional performance and they have become widely used for reasons including their excellent sensitivity, dynamic range, precision, and tolerance of complex sample matrices. Species that can be induced to emit ECL (ECL-active species) have been used as ECL labels, e.g., (i) organometallic compounds where the metal is from, for example, the noble metals of group VIII, including Ru-containing and Os-containing organometallic compounds such as the tris-bipyridyl-ruthenium (RuBpy) moiety, and (ii) luminol and related compounds. Species that participate with the ECL label in the ECL process are referred to herein as ECL coreactants. Commonly used coreactants include tertiary amines (e.g., see U.S. Patent No. 5,846,485), oxalate, and persulfate for ECL from RuBpy and hydrogen peroxide for ECL from luminol (see, e.g., U.S. Patent No. 5,240,863). The light generated by ECL labels can be used as a reporter signal in diagnostic procedures (Bard et al., U.S. Patent No. 5,238,808, herein incorporated by reference). For instance, an ECL label can be covalently coupled to a binding agent such as an antibody, nucleic acid probe, receptor or ligand; the participation of the binding reagent in a binding interaction can be monitored by measuring ECL emitted from the ECL label. Alternatively, the ECL signal from an ECL-active compound may be indicative of the chemical environment (see, e.g., U.S. Patent No. 5,641,623 which describes ECL assays that monitor the formation or destruction of ECL coreactants). For more background on ECL, ECL labels, ECL assays and instrumentation for conducting ECL assays see U.S. Patents Nos. 5,093,268; 5,147,806; 5,324,457; 5,591,581; 5,597,910; 5,641,623; 5,643,713; 5,679,519; 5,705,402; 5,846,485; 5,866,434; 5,786,141; 5,731,147; 6,066,448; 6,136,268; 5,776,672; 5,308,754; 5,240,863; 6,207,369; 6,214,552 and 5,589,136 and Published PCT Nos. WO99/63347; WO00/03233; WO99/58962; WO99/32662; WO99/14599; WO98/12539; WO97/36931 and WO98/57154, all of which are incorporated herein by reference.

In certain embodiments, plates adapted for use in electrochemiluminescence (ECL) assays are employed as described in U.S. Applications 10/185,274; 10/185,363; and 10/238,391. In assay methods that detect ECL from one well at a time, the electrode and electrode contacts in these wells are adapted to allow application of electrical energy to electrodes in only one well at a time. The apparatus may be particularly well-suited for carrying out assays in plates containing dry reagents and/or sealed wells, e.g., as described in U.S. Application 11/642,970 of Glezer et al.

In one embodiment, the invention provides a method for conducting a measurement using a multi-well assay test plate and a multi-well auxiliary plate. That method may include the following steps:
(a) dispensing a sample and/or a reagent into a first auxiliary well of the auxiliary plate; and
(b) transferring the sample and/or reagent from the auxiliary well to a first test well of a the assay test plate.

The method may further comprise repeating steps (a) and (b) in additional auxiliary and test wells. The steps may be repeated using the same sample and/or reagent or different samples and/or reagents.

A sample and a reagent may both be dispensed into an auxiliary well to dilute the sample prior to further analysis in the assay test plate. The auxiliary wells into which a sample and/or a reagent are dispensed may contain liquid or dried auxiliary reagents which are mixed or reconstituted into the dispensed samples and/or reagents.

Dispensing step (a) may be used as a pre-treatment step to prepare a sample or reagent for analysis in the assay test plate (for example, by diluting the sample, providing conditions that optimally present a biomarker in the sample, providing an assay matrix appropriate for the assay measurement, providing assay reagents used in the assay measurement, etc.). The step may be used to reconstitute, in the dispensed sample and/or reagent, a dried assay reagent that is provided in the auxiliary plate. Accordingly, transfer step (b) may comprise transferring the pre-treating sample and/or reagent and/or the reconstituted dried reagent from the auxiliary well to the well of the test plate.

In one embodiment of the invention, a method for conducting a measurement using a multi-well assay plate and a multi-well auxiliary plate involves sequentially transferring a sample and/or a reagent to a plurality of different auxiliary wells in the auxiliary plate (e.g., transferring the sample and/or reagent to a first well, and then from the first well to a second well, then from the second well to a third well, etc.). Such a process may be carried out, for example, to serially dilute the sample and/or reagent, to expose a sample to reactive conditions needed to extract/present a biomarker and then to quench/neutralize the reactive conditions prior to transferring the sample to the assay plate and/or to reconstitute multiple dried auxiliary reagents into the sample and/or reagent. The method may include the following steps:
(a) dispensing a sample and/or a reagent into a first auxiliary well of a first set of auxiliary wells in the auxiliary plate;
(b) transferring the sample and/or reagent from the first auxiliary well of the first set to a second auxiliary well of the first set;
(c) optionally, sequentially transferring the sample to one or more additional auxiliary wells of the first set; and
(b) transferring the sample and/or reagent that has undergone the transfer steps of step (b) or step (c), if optional step (c) was carried out, to a first test well of the assay test plate.

The method may further comprise repeating steps (a) and (b) in additional test wells and sets of auxiliary wells. The steps may be repeated using the same sample and/or reagent or different samples and/or reagents.

In one embodiment, the one, two or more assay test plates are supported on a plate translation stage and the method comprises translating the test plate(s) via the plate translation stage (see, e.g., plate translation stage 230 of Figure 3). During the processing of an assay well, the method may further comprise translating the plate translation stage to one or more pre-defined processing positions that align the well with certain components of the apparatus. By way of example, there may be a pipetting position in which the well may be accessed by the instruments pipettors, a washing position in which the well may be accessed by the instruments washing probe and an analysis position in which the well is aligned with signal induction and/or detection components (e.g., in the case of instrumentation for ECL assays, an electrical contact mechanism and an ECL imaging component. The method may further include (i) lowering a first assay test plate from the input plate stacker to a first assay plate location on the plate translation stage, (ii) translating the plate translation stage to one or more pre-defined processing positions for one or more wells of the first assay test plate, and (iii) raising the first assay test plate from the plate translation stage to an output plate stacker. Optionally, the plate translation stage comprises a second assay plate location, step (i) further comprises lowering a second assay test plate from the input plate stacker to a second assay plate location on the plate location stage, step (ii) further comprises translating the plate translation stage to one or more pre-defined processing positions for one or more wells of the second assay test plate and step (iii) further comprises raising the second assay test plate from the plate translation stage to an output plate stacker.

The apparatus used in the methods of the invention may be configured to enable replacement of used test or auxiliary plates without interrupting the ability of the instrument to accept and process new samples. Accordingly, the methods may comprise processing samples using wells of a first assay test plate (or auxiliary plate), determining when all the wells of the first assay test plate (or auxiliary plate) have been committed, processing additional samples using wells of a second assay test plate (or auxiliary plate). The methods may further comprise, determining when processing of the first assay test plate (or auxiliary plate) is complete, replacing the first assay test plate (or auxiliary plate) with a third assay test plate (or auxiliary plate), determining when all the wells of the second assay test plate (or auxiliary plate) have been committed and processing additional samples using wells of the third assay test plate (or auxiliary plate). Such a sequence can be used indefinitely to replace plates as they are used up, without interrupting the sample processing workflow. In one embodiment an instrument of the invention (e.g., the instrument of Figure 1(a)), is used to carry out an assay method comprising:
(i) lowering a first multi-well assay test plate from an input plate stacker to a first assay plate location on the plate translation stage,
(ii) lowering a second multi-well assay test plate from an input plate stacker to a second assay plate location on the plate translation stage,
(iii) locking first and a second auxiliary plates into first and second auxiliary plate locations, wherein the auxiliary plates include a number of sets of auxiliary wells that corresponds to the number of test wells in the assay test plate,
(iv) processing samples using the wells of the first assay test plate and the sets of the first auxiliary plate,
(v) determining when all wells and sets of the first test and auxiliary plates have been committed to samples,
(vi) processing additional samples using the wells of the second assay test plate and the sets of the second auxiliary plate,
(vii) determining when processing of the first test and auxiliary plates is complete and raising the first test plate to an output plate stacker and releasing the first auxiliary plate,
(viii) lowering a third multi-well assay test plate from the input plate stacker to the first assay plate translation stage
(ix) locking a third auxiliary plate into the first auxiliary plate location,
(x) determining when all wells and sets of the second test and auxiliary plates have been committed to samples
(v) processing further additional samples using the wells of the third assay test plate and the sets of the third auxiliary plate.

In the method described immediately above, the number of wells in the assay test plate is matched to the number of sets of wells in the auxiliary plate so that both consumables are used at the same rate and may be replaced at the same time. In an alternative embodiment, the number of sets of wells in the auxiliary plate may be a multiple (e.g., 2, 3, 4, etc.) or even factor (e.g., 2, 3, 4, etc.) of the numbers of test wells in the test plate. In this case, the auxiliary plate would be replaced with every nth (e.g., every second, third of fourth) test plates or the test plate would be replaced with every nth (e.g., every second, third or fourth) auxiliary plate. In another alternative embodiment, the numbers of test wells and sets of auxiliary wells per plate are set at any specific ratio and the method provides independent plate usage monitoring and plate replacement for test and auxiliary plates.

One approach to measuring multiple samples in an automated instrument is a serial sample process that involves completing sample analysis for one sample before beginning analysis on the next sample, a process that provides a low sample-throughput. The serial approach is generally not time efficient, especially if specific instrument components (pipettors, imaging subsystems, etc.) are only active during brief periods during the analysis of a sample. To increase throughput, it is possible to begin processing for a sample while processing of previous samples is in progress by an interleaved scheduling approach that takes advantage of the periods of time when specific components are not being used to process the previous samples.

One embodiment of such an approach is a continuous interleaved process that is based on a repeating block of processing steps. The block is further broken down into time slices that are dedicated to the different actions the instrument carries out during a sample processing sequence. During a time slice, the instrument carries out the actions associated with the time slice if there is a sample at the correct stage in the assay process to receive the action, otherwise no action is taken during the time slice. By way of example, an assay process may have a dedicated sample addition time slice during which the instrument pipettor transfers sample from a sample tube to a test or auxiliary well. If a sample is available in the sample queue, the action will take place. If there is no sample in the queue, the pipettor will sit idle for that time slice. Similarly, the assay process may have a dedicated test well wash time slice during which a well that has reached the end of an incubation phase will be washed prior to further processing or analysis. If no sample has reached the end of an incubation phase during a given block, the well washing components will remain idle during that time slice. To gain further time efficiencies, the time slices may overlap, for example, if actions in different time slices use components that can operate independently of each other. There may also be time slices that do not overlap within a block, particularly if the time slices both require the dedicated use of the same instrument component.

Preferably, an instrument running a continuous interleaved process, as described above, includes computer control with a software scheduler that tracks the status of all the assays running on the instrument at any given time which wells or samples, if any, are acted on during a specific time slice in a specific process block. The scheduling approach described above ensures that all wells are processed using substantially the same assay protocol and timing while following a fairly simple scheduling algorithm. Alternatively, the software scheduler may be programmed to adjust one or more of the steps in the protocol, as determined by the user.

A processing block may comprise, but is not limited to, one or more time slices selected from the group consisting of:
(a) one or more auxiliary plate sample addition phases, wherein the pipetting subassembly is engaged to transfer sample from a sample tube to a well of an auxiliary plate and wherein the pipetting subassembly may be further engaged to pierce a seal on the well, add a diluent to the well and/or to mix the contents of the well;
(b) one or more auxiliary plate sample transfer phases, wherein the pipetting subassembly is engaged in transferring a sample from a first well of an auxiliary plate to a second well of the auxiliary plate, and wherein the pipetting subassembly may be further engaged to pierce a seal on, add a diluent to and/or to mix the contents of the second well;
(c) one or more reagent reconstitution phases, wherein the pipetting subassembly is engaged in adding a diluents to a well of an auxiliary plate and wherein the pipetting subassembly may be further engaged pierce a seal on the well and/or to mix the contents of the well;
(d) one or more test plate addition phases, wherein the pipetting subassembly is engaged in transferring a reagent or sample from a sample tube or auxiliary well to a test well of an assay test plate;
(e) one or more assay well wash phases, wherein the wash subassembly is engaged in washing a well of an assay test plate, and wherein the wash subassembly may be further engaged to add a read buffer to the well;
(f) one or more detection phases, wherein the detection subassembly is engaged in detecting and, optionally, inducing an assay signal from a well of an assay test plate;
(g) one or more assay well piercing phases, wherein the wash subassembly is engaged in piercing a well of an assay test plate;
(h) one or more assay test plate shaking phases, wherein the plate translation stage is engaged to shake assay test plates held on the stage
(i) one or more consumable identifier information transfer phases, wherein an identifier on a consumable is read or updated; and
(i) one or more instrument preparation/maintenance phases which may include fluidics priming phases, probe cleaning phases, plate loading and unloading phases, etc.

The invention includes an assay process in which assay operations (which may include one or more of the assay operations described above) are accorded time slices within a repeating sequence of process blocks (blocks 1, 2, 3, etc.), the process comprising:
(a) carrying out a first set of assay operations in process block 1 for the analysis of a first sample, wherein the first set of assay operations are accorded a first set of time slices within each process block;
(b) carrying out a second set of assay operations in process block 1+n for the analysis of the first sample, wherein the second set of assay operations are accorded a second set of time slices within each process block and n is an integer ≥ 1;
(c) carrying out the first set of assay operations for the analysis of a second sample during process block 1+x, wherein m is an integer and 1 < x ≤ n; and
(d) carrying out the second set of assay operations for the analysis of the second sample during process block 1+x+n.

The assay process may further additional assay operations for each sample carried out in additional time slices in additional process blocks. For example, the analysis of the first sample may further comprise a third set of assay operations in process block 1+n+m and/or a fourth set of assay operations in process block 1+n+m+o and so on, where m and o are integers ≥ 1 and each set of assay operations are according their dedicated time slices within a processing block. By analogy, the analysis of the second sample would further comprise carrying out the third and/or fourth set of operations in blocks 1+x+m and 1+x+o.

The assay process may also further comprise carrying out the assay operations on additional samples (or, carrying out replicate analyses on the same sample), for example, analysis of third and/or fourth samples could be carried out by carrying out the first set of assay operations for these samples in process blocks 1+x+y and 1+x+y+z, where y and z are integers ≥ 1 and the subsequent operations are on each sample are carried out using the same timing as for the first and second samples. In one embodiment of the invention, the first set of assay operations is carried out for a new sample (or for a replicate of an old sample) during each of a continuous series of a plurality of process blocks, for example, for a series of 2, 3, 10, 48, 49, 96, 97, 192, 193 or more process blocks. As previously described, if a sample is not present that requires a specific processing action during a specific processing block, that processing action may be omitted. Preferably, the instrument scheduler is configured to allow for omission of the action, without affecting the duration of the processing block or the timing of other actions within the processing block.

The assay process may comprise an operation in that involves creating an assay reaction mixture (e.g., by combining a sample with a reagent in an auxiliary well or an assay test well) and an operation that involves analysis or further processing of the product of the assay reaction (e.g., washing a well, inducing and/or detecting an assay, transferring a reaction product from an auxiliary well to another auxiliary well or an assay test well). Both operations may occur at defined times within process blocks. Alternatively, the assay reaction mixture may be created in an initial processing block (e.g., process block 1) and the product analyzed/processed in a subsequent processing block (e.g., processing block 1+n). Selection of the number of intervening blocks between the initial and subsequent block (the value of n) provides a configurable approach to setting the incubation time for the assay reaction. The process blocks of the assay process may comprise one or more assay test plate shaking phases, which may be used to mix solutions in the test plates and accelerate assay reactions in the test plates. In one embodiment of the invention, the assay process is configured such that when samples are being processed in an assay plate, each process block includes a substantially identical series of shaking phases, whether or not other process operations are occurring in these blocks (e.g., during process blocks when samples are being incubated, but no samples are present that require other operations). This approach can be used to eliminate significant variability in assay signals due to variations in the shaking profiles observed, for each sample, in assay reactions carried out in assay test plates.

Certain instrument maintenance operations (e.g., priming of fluidic lines or replacement of assay test plates, auxiliary plates and/or other consumables) may occur at relatively infrequent intervals and, e.g., may not be required to occur with every sample or in every process block. A continuous interleaved assay process (as described above) may include one or more of such maintenance operations, including replacement of assay test plates, while maintaining continuous operation.

In one embodiment of an assay process that includes maintenance operations, every process block includes time slices for these maintenance operations; the software scheduler may schedule the operations in some process blocks and omit them from others according based on instrument usage or a maintenance schedule.

In an alternate embodiment of a process that includes maintenance operations, the normal process block is replaced with a maintenance process block when certain maintenance functions are required (e.g., replacement of assay test plates and/or other consumables). In the maintenance process block, the time slices associated with beginning processing of a new sample are omitted and replaced with time slices associated with the maintenance operations. If there are samples waiting in the sample queue, initiation of processing of these samples is bumped back one process block. In this embodiment, the maintenance process block is designed such that it has the same duration as the normal process block and such that the timing for operations on in-process samples is not affected.

In another alternate embodiment of a process that includes maintenance operations, when the maintenance operations are required, the normal process block is replaced with a maintenance process block in which the time slices associated with processing samples are omitted and replaced with time slices associated with the maintenance operations (optionally, time slices associated with controlled incubation of samples, such as plate shaking, are kept). The maintenance process block is designed such that it has the same duration as the normal process block and such that any assays that are in an incubation phase during the block are not affected. In one approach to scheduling the maintenance operations in such a maintenance block, the software scheduler determines in advance when the maintenance block will be run and does not introduce new samples into any prior blocks if those samples would require a processing operation (other than shaking) during the scheduled maintenance block. In addition, the scheduler may, based on the sample queue and the status of in-process samples, determine that there is an open process block in which no samples require processing (other than shaking) and insert a maintenance block into that time slot. In a different scheduling approach, every k^{th} process block is pre-scheduled as a maintenance block (where k is any integer, optionally between 4 and 24 or between 6 and 12). In any one of these given maintenance blocks, specific maintenance functions may be carried out or omitted according to the maintenance requirements. In this approach, the assay operations associated with a specific sample (other than shaking) are spaced by multiples of k to ensure that no sample processing is required during the maintenance blocks (e.g., if k is 6, processing of a sample may begin in block 1, and additional processing step may occur in block 7 and a final processing step may occur in block 31).

The invention includes an assay process in which assay operations are accorded time slices within a repeating sequence of process blocks (blocks 1, 2, 3, etc.), the process comprising:
(a) carrying out a first set of assay operations for a first sample in process block 1 comprising
   (i) transferring the first sample from a sample tube to a first well of a first set of auxiliary wells in an auxiliary plate and combining the first sample with an assay reagent (which may be a dried reagent provided in the auxiliary well);
   (ii) mixing the first sample in the auxiliary well;
(b) carrying out a second set of assay operations for the first sample in a subsequent process block (block 1+n, where n is an integer ≥ 1) comprising
   (i) transferring the first sample from the first well of the first set of auxiliary wells to a second auxiliary well of the first set of auxiliary wells and combining the sample with an assay reagent (which may be a dried reagent provided in the auxiliary well);
   (ii) mixing the first sample in the auxiliary well;
   (iii) transferring the first sample to a first assay well in an assay test plate;
(c) carrying out a third set of assay operations for the first sample in a subsequent process block (block 1+n+m, where m is an integer ≥ 1) comprising
   (i) dispensing an assay diluent into a third well of the first set of auxiliary wells and reconstituting a dry assay reagent in the well;
   (ii) washing the first assay well (e.g., by one or more cycles of aspirating the contents of the well and dispensing a wash buffer to the well);
   (iii) aspirating the contents of the first assay well;
   (iv) transferring the reconstituted dry reagent from the third well of the first set of auxiliary wells to the first assay well;
(d) carrying out a third set of assay operations for the first sample in a subsequent process block (block 1+n+m+o, where o is an integer ≥ 1) comprising
   (i) washing the first assay well (e.g., by one or more cycles of aspirating the contents of the well and dispensing a wash buffer to the well);
   (ii) optionally, aspirating the contents of the first assay well and dispensing a detection buffer (e.g., an ECL read buffer) to the well;
   (iii) inducing and/or detecting an assay signal from the first assay well;
(e) carrying out the steps (a)-(d) for one or more additional samples, initiating each of the one or more additional samples in a different process block, and using a different assay wells and a different set of auxiliary wells for each sample.

The invention also includes alternative embodiments that have one or more of the following modifications:
(1) the operations in steps (a) and (b) may be carried out in one process block (in which case n=0);
(2) the operations in step (b) may be omitted (in which case n=0) and step (a) further comprises (iii) transferring the first sample to a first assay well in an assay test plate;
(3) the operations in step (c) are omitted (in which case m=0);
(4) the sets of operations in steps (a)-(d) are carried out on two or more samples in each process block, using two or more additional assays wells and sets of auxiliary wells (e.g., step (a)(i) may further comprise transferring a second sample from a second sample tube to a first well of a second set of auxiliary wells, and so on); and/or
(5) the sets of operations in steps (a)-(d) for a sample are repeated in additional assay wells and sets of auxiliary wells (e.g., step (a)(i) may further comprise transferring the first sample to a first well of a second set of auxiliary wells and step(b)(iii) may further comprise transferring the first sample from the second set of auxiliary wells to a second assay well).

The process, optionally, also includes replacement of used assay test plates and/or auxiliary plates and may include carrying out a maintenance block as described above.

In one embodiment, the method includes (a) introducing a sample tube rack into the sample rack subassembly; (b) reading sample and assay-specific information from the identifiers on the sample rack subassembly and/or reading sample and assay-specific information manually input into the computer user interface by the user; (c) introducing an auxiliary plate to the auxiliary plate subassembly; (d) reading assay-specific information from the identifiers on the auxiliary plate; (e) introducing a test plate into the plate introduction aperture of the light-tight enclosure; (f) reading assay-specific information from the identifiers on the test plate; (g) sealing the door of the plate introduction aperture, (f) translating the test plate to position one or more wells under the light detector, (g) rehydrating reagents in one or more auxiliary wells of the auxiliary plate using the pipetting arm subassembly and/or pretreating one or more wells of the test plate using the pipetting arm subassembly; (h) collecting a sample volume from a sample tube of the sample rack subassembly and pipetting that sample volume into a well of an assay test plate; (i) collecting sample reagents from the auxiliary plate and dispensing those reagents into a well of the assay test plate; (j) detecting luminescence from the one or more wells, (k) repeating one or more of the preceding steps on additional wells of the test plate, using additional auxiliary wells of the auxiliary plate; (j) translating the used test plate to a plate elevator; (k) raising the plate elevator; and (1) removing the test plate from the plate introduction aperture.

The method may also, optionally, comprise one or more of: (i) pre-treating sample and/or reagent in a auxiliary well of the auxiliary plate and pipetting that pre-treated sample and/or reagent into or out of one of a auxiliary well of a test plate; (ii) removing seals from one or more of the auxiliary wells and/or wells of the auxiliary plate and/or test plate, respectively, or (iii) applying electrical energy to electrodes in one or more of the test plate wells (e.g., to induce electrochemiluminescence).

The apparatuses, consumables and methods may be used for conducting assays on clinical samples. They may be particularly well-suited for conducting automated sample preparation and analysis in the multi-well plate assay format. The biological agents that may be detected include viral, bacterial, fungal, and parasitic pathogens as well as biological toxins. The agents themselves may be detected or they may be detected through measurement of materials derived from the agents including, but not limited to, cellular fragments, proteins, nucleic acids, lipids, polysaccharides, and toxins.

The apparatus and assay consumables described herein may be used to carry out panels of assays. Panels of analytes that can be measured in a sample include, for example, panels of assays for analytes or activities associated with a disease state or physiological conditions. Certain such panels include panels of cytokines and/or their receptors (e.g., one or more of TNF-alpha, TNF-beta, ILl-alpha, ILl-beta, IL2, IL4, IL6, IL-10, IL-l2, IFN-y, etc.), growth factors and/or their receptors (e.g., one or more of EGF, VGF, TGF, VEGF, etc.), drugs of abuse, therapeutic drugs, vitamins, pathogen specific antibodies, auto-antibodies (e.g., one or more antibodies directed against the Sm, RNP, SS-A, SS-alpha, J0-1, and Scl-70 antigens), allergen-specific antibodies, tumor markers (e.g., one or more of CEA, PSA, CA-125 II, CA 15-3, CA 19-9, CA 72-4, CYFRA 21-1, NSE, AFP, etc.), markers of cardiac disease including congestive heart disease and/or acute myocardial infarction (e.g., one or more of Troponin T, Troponin I, myoglobin, CKMB, myeloperoxidase, glutathione peroxidase, β-natriuretic protein (BNP), alpha-natriuretic protein (ANP), endothelin, aldosterone, C-reactive protein (CRP), etc.), markers associated with hemostasis (e.g., one or more of Fibrin monomer, D-dimer, thrombin-antithrombin complex, prothrombin fragments 1 & 2, anti-Factor Xa, etc.), markers of acute viral hepatitis infection (e.g., one or more of IgM antibody to hepatitis A virus, IgM antibody to hepatitis B core antigen, hepatitis B surface antigen, antibody to hepatitis C virus, etc.), markers of Alzheimers Disease (alpha-amyloid, beta-amyloid, Aβ 42, Aβ 40, Aβ 38, Aβ 39, Aβ 37, Aβ 34, tau-protein, etc.), markers of osteoporosis (e.g., one or more of cross-linked Nor C-telopeptides, total deoxypyridinoline, free deoxypyridinoline, osteocalcin, alkaline phosphatase, C-terminal propeptide of type I collagen, bone-specific alkaline phosphatase, etc.), markers of fertility state or fertility associated disorders (e.g., one or more of Estradiol, progesterone, follicle stimulating hormone (FSH), lutenizing hormone (LH), prolactin, hCG, testosterone, etc.), markers of thyroid disorders (e.g., one or more of thyroid stimulating hormone (TSH), Total T3, Free T3, Total T4, Free T4, and reverse T3), and markers of prostrate cancer (e.g., one or more of total PSA, free PSA, complexed PSA, prostatic acid phosphatase, creatine kinase, etc.). Certain embodiments of invention include measuring, e.g., one or more, two or more, four or more or 10 or more analytes associated with a specific disease state or physiological condition (e.g., analytes grouped together in a panel, such as those listed above; e.g., a panel useful for the diagnosis of thyroid disorders may include e.g., one or more of thyroid stimulating hormone (TSH), Total T3, Free T3, Total T4, Free T4, and reverse T3).

Preferred panels also include nucleic acid arrays for measuring DNA or RNA levels. Such arrays may be used to detect, e.g., nucleic acids associated with the presence of specific organisms or pathogens, the presence of specific disease states, specific genotypes, etc. In one embodiment, such arrays are used to measure mRNA levels of mRNA coding for cytokines, growth factors, components of the apoptosis pathway, expression of the P450 enzymes, expression of tumor related genes, pathogens (e.g., the pathogens listed above), etc. Preferred panels also include nucleic acid arrays for genotyping individuals (e.g., SNP analysis), pathogens, tumor cells, etc. Preferred panels also include libraries of enzymes and/or enzyme substrates (e.g., substrates and/or enzymes associated with ubiquitination, protease activity, kinase activity, phosphatase activity, nucleic acid processing activity, GTPase activity, guanine nucleotide exchange activity, GTPase activating activity, etc.). Preferred panels also include libraries of receptors or ligands (e.g., panels of G-protein coupled receptors, tyrosine kinase receptors, nuclear hormone receptors, cell adhesion molecules (integrins, VCAM, CD4, CD8), major histocompatibility complex proteins, nicotinic receptors, etc.). Preferred panels also include libraries of cells, cell membranes, membrane fragments, reconstituted membranes, organelles, etc. from different sources (e.g., from different cell types, cell lines, tissues, organisms, activation states, etc.).

A method is also provided for conducting assays for biological agents. In one embodiment, the method is a binding assay. In another embodiment, the method is a solid-phase binding assay (in one example, a solid phase immunoassay) and comprises contacting an assay composition with one or more binding surfaces that bind analytes of interest (or their binding competitors) present in the assay composition. The method may also include contacting the assay composition with one or more detection reagents capable of specifically binding with the analytes of interest. The multiplexed binding assay methods according to preferred embodiments can involve a number of formats available in the art. Suitable assay methods include sandwich or competitive binding assays format. Examples of sandwich immunoassays are described in U.S. Patents 4,168,146 and 4,366,241. Examples of competitive immunoassays include those disclosed in U.S. Patents 4,235,601; 4,442,204; and 5,208,535 to Buechler et al. In one example, small molecule toxins such as marine and fungal toxins can be advantageously measured in competitive immunoassay formats.

Binding reagents that can be used as detection reagents, the binding components of binding surfaces and/or bridging reagents include, but are not limited to, antibodies, receptors, ligands, haptens, antigens, epitopes, mimitopes, aptamers, hybridization partners, and intercalaters. Suitable binding reagent compositions include, but are not limited to, proteins, nucleic acids, drugs, steroids, hormones, lipids, polysaccharides, and combinations thereof. The term "antibody" includes intact antibody molecules (including hybrid antibodies assembled by *in vitro* re-association of antibody subunits), antibody fragments, and recombinant protein constructs comprising an antigen binding domain of an antibody (as described, e.g., in Porter & Weir, J.Cell Physiol., 67 (Suppl 1):51-64, 1966; Hochman et al., Biochemistry 12:1130-1135, 1973; hereby incorporated by reference). The term also includes intact antibody molecules, antibody fragments, and antibody constructs that have been chemically modified, e.g., by the introduction of a label.

Measured, as used herein, is understood to encompass quantitative and qualitative measurement, and encompasses measurements carried out for a variety of purposes including, but not limited to, detecting the presence of an analyte, quantitating the amount of an analyte, identifying a known analyte, and/or determining the identity of an unknown analyte in a sample. According to one embodiment, the amounts the first binding reagent and the second binding reagent bound to one or more binding surfaces may be presented as a concentration value of the analytes in a sample, i.e., the amount of each analyte per volume of sample.

Analytes may be detected using electrochemiluminescence-based assay formats. Electrochemiluminescence measurements are preferably carried out using binding reagents immobilized or otherwise collected on an electrode surface. Especially preferred electrodes include screen-printed carbon ink electrodes which may be patterned on the bottom of specially designed cartridges and/or multi-well plates (e.g., 24-, 96-, 384- etc. well plates). Electrochemiluminescence from ECL labels on the surface of the carbon electrodes is induced and measured using an imaging plate apparatus as described in copending U.S. Applications 10/185,274 and 10/185,363 (both entitled "Assay Plates, Apparatus Apparatus' and Methods for Luminescence Test Measurements", filed on June 28, 2002, hereby incorporated by reference). Analogous plates and plate apparatus' are now commercially available (MULTI-SPOT® and MULTI-ARRAY® plates and SECTOR® instruments, Meso Scale Discovery, a division of Meso Scale Diagnostics, LLC, Gaithersburg, MD).

In one embodiment, antibodies that are immobilized on the electrodes within the plates may be used to detect the selected biological agent in a sandwich immunoassay format. In another embodiment, microarrays of antibodies, patterned on integrated electrodes within the plates, will be used to detect the plurality of the selected biological agents in a sandwich immunoassay format. Accordingly, each well contains one or more capture antibodies immobilized on the working electrode of the plate and, optionally, in dry form, labeled detection antibodies and all additional reagents necessary for analysis of samples, and for carrying out positive and negative controls. In one example, arrays having multiple binding surfaces within a single well allow tests to be replicated to significantly reduce false positive identification.

A positive control method is provided to identify conditions or samples that may cause false negative measurements by interfering with the generation of signal. According to this aspect, positive control method comprises contacting sample with a binding reagent (e.g., an antibody) to a positive control substance (for example, to a nontoxic positive control substance) that is not expected to be observed in environmental samples; then contacting the sample with a labeled detection reagent (for example, an antibody) against the positive control substance and a controlled amount of the positive control substance, and measuring the signal. The positive control should, therefore, always provide a constant positive signal regardless of the sample. A significantly reduced signal may indicate that the sample interferes with the antibody binding reactions or the signal generating process, or may indicate a malfunction in the plate or instrument.

A negative control method is provided employing a capture reagent (e.g., an antibody) that is not matched with a detection reagent. The method comprises contacting a sample with a capture reagent in the presence of mismatched detection reagent and measuring signal. The negative control should, therefore, provide a negative signal regardless of the sample. A significantly elevated signal from the negative control indicates the presence of a material in the sample, such as a cross-linking agent, that is causing the non-specific binding of non-matched detection reagents to the negative control capture reagent.

A method is provided using a mixture of non-specific antibodies from the same species (e.g., polyclonal mouse, rabbit, goat, etc.) as specific capture antibodies to identify any non-specific binding effects that would otherwise provide false positive identification. This mixture may be selected to include the species of the antibodies used in the actual test measurements.

A method is provided using at least two different pairs of capture and detection reagents (e.g., antibodies) in alternating independently addressable wells to reduce the frequency of false positive identifications. Accordingly, the first binding reagent pair is used as a primary identification, which, if positive, triggers the confirmation test using the second binding reagent pair. The pairs may target the same marker or epitopes of a biological agent or, alternatively, they may further increase the orthogonality of the two measurements by targeting different markers or epitopes of a biological agent. An arrangement of at least two different antibody pairs in alternating well may be particularly advantageous. According to this aspect, the pairs are alternating as a primary identification set, thereby eliminating the need to dedicate wells as confirmation tests. Instead, if a sample is suspected to be positive based on the most recent test (based on either the first or the second pair), confirmation is simply performed by running the subsequent test well.

The reliability of detection method may be further improved by providing two or more different capture antibodies in a single well, wherein (a) the two or more different antibodies recognize the same marker and/or epitope of the same biological target; and/or b) the two or more different antibodies recognize different markers and/or epitopes of the same biological target.

In one embodiment, the plate has an immobilized array of binding reagents (e.g., antibodies or nucleic acids) and bioagents in the sample bind to the corresponding immobilized reagent and a corresponding labeled detection reagent to form a sandwich complex. In some, the array is formed on an electrode and detection is carried out using an ECL measurement. In one embodiment, after addition of an ECL read buffer, labels on the electrode are induced to emit ECL by applying a voltage to the working electrode, and the emitted ECL is imaged with a CCD camera. Optionally, washing may be added prior to the ECL measurement to provide advantages in assay sensitivity, particularly for optically turbid samples generated by aerosol samplers in dirty environments. Image analysis is used to determine the location of the emitted light on the array and, thus, the identity of the agents in the sample. Image analysis also provides the intensity of the emitted light from each element of the antibody array and allows for precise quantitation of each bioagent.

The apparatus of the present invention employs a variety of assay consumables, e.g., sample tubes, tube racks, auxiliary plates, test plates, and liquid reagent compartments. In one embodiment, the assay consumable comprises an identifier (referred to alternatively throughout the specification as an identifier, a consumable identifier, or an assay consumable identifier) and the assay apparatus or a component thereof comprises an identifier controller that interacts with the identifier. As described herein, the identifier includes information concerning the assay consumable, which may include but is not limited to, how the consumable is manufactured and handled prior to use and how the consumable is used in an apparatus. Therefore, the apparatus is configured to use an assay consumable in the conduct of an assay, and the apparatus includes an apparatus adapted to perform an operation selected from (i) reading information from an assay consumable identifier associated with the assay consumable; (ii) erasing information from the assay consumable identifier; and/or (iii) writing information to the assay consumable identifier. The information may be used by the apparatus to perform a variety of operations, e.g., to perform any aspect of a biological assay, tracking the use and/or performance of the assay consumable and/or the assay apparatus, associating particular information unique to that assay consumable with that consumable so that the information may be accessed and used in subsequent applications in the same or a different assay apparatus, and/or to adjust one or more operations performed by the apparatus before, during and/or after the conduct of an assay by the apparatus. Regarding the use of assay consumable identifiers to track usage and manufacturing in assay apparatus', reference is made to copending U.S. Provisional Patent Application Serial No. 61/271,873, filed July 27, 2009 (Ref. No. 221000USPR00), the disclosure of which is incorporated herein by reference in its entirety.

In one embodiment, the assay consumable identifier comprises memory for storing information related to the consumable, its history and/or its use. In one embodiment, the memory is non-volatile memory. Non-volatile memory is computer memory that can retain the stored information without power. In one embodiment, the non-volatile memory used in the present invention is selected from the group consisting of an EEPROM, bar code, flash memory, ICC and combinations thereof. In one embodiment, the non-volatile memory is an EEPROM. In an alternate embodiment, the non-volatile memory is an RFID. In a further embodiment, the non-volatile memory is a bar code.

In an additional alternative embodiment, two or more non-volatile memory components may be used in the present invention. For example, a first assay consumable comprising a first identifier may be used in the assay apparatus, and an additional assay consumable comprising an additional identifier may also be used in the assay apparatus. Each identifier may include the same or different type of memory. However, for each different form of memory, there will be a separate identifier controller. And certain assay information may be stored on one identifier and other assay information on an additional identifier of the same or different type. For example, one assay consumable used in the apparatus may comprise an EEPROM or RFID as an identifier, whereas the apparatus may also use an additional assay consumable comprising, e.g., a bar code as a identifier. The assay apparatus would comprise an identifier controller capable of interfacing with the first identifier, i.e., the EEPROM or RFID, and the apparatus will further comprise an additional controller that will interface with the bar code.

The assay apparatus of the present invention includes an identifier controller that controls the operation of the non-volatile memory and other components of the assay apparatus. The identifier controller optionally includes a micro-controller to interface with the non-volatile memory over a communication interface, which may incorporate conventional interface architectures and protocols such as I²C, a two line serial bus protocol. The microcontroller addresses the non-volatile memory and performs write, read and erase operations on the memory.

The consumable identifier may be located on the consumable or it may be a separate component. In either case, the apparatus may be designed to have a unique identifier for each consumable. Alternatively, the apparatus may be configured so that one separate consumable identifier is used to hold information relating to a plurality of consumables. In one example, each package of consumables has a package-specific identifier mounted on the package (or, alternatively, supplied in the package) that holds information relating to the plurality of consumables in the package. Optionally, each consumable also carries an additional unique consumable-specific identifier attached to the consumable. This consumable-specific identifier is used primarily to uniquely identify the consumable and link it to information on the package-specific identifier. In this embodiment, lot information content and/or non-editable identifiers such as bar codes may be used.

The identifier is programmed, e.g., during the manufacturing process or at another time prior to use in the assay apparatus. The identifier may be programmed with information (referred to alternatively herein as "assay information" or "assay consumable information") which is used before, during or after an assay or a step of a multi-step assay to control the operation of the assay apparatus, apparatus or a component of the assay apparatus. The term "assay information" may include any information used to uniquely identify a particular assay or assay step, assay consumable, consumable domain(s), biological reagent or sample or to distinguish a particular assay, assay step, assay consumable, consumable domain(s), biological reagent or sample from other assay consumables, consumable domains, biological reagents or samples. Assay information may include consumable information, sample information, chain of custody information, consumable/test well information, assay process information, consumable security information, and combinations thereof. Each type of assay information is described in more detail below.

For example, the assay information may include consumable information that includes but is not limited to lot identification information, lot specific analysis parameters, manufacturing process information, raw materials information, expiration date, Material Safety Data Sheet (MSDS) information, product insert information (i.e., any information that might be included or described in a product insert that would accompany the assay consumable, e.g., the assay type, how the assay is performed, directions for use of the assay consumable, assay reagents, or both, etc.), threshold and/or calibration data for one or more reagents used in the assay consumable or in an assay or a step of a multi-step assay, and the location of individual assay reagents and/or samples within one or more test wells of the assay consumable.

The consumable identifier may also include lot identification information, i.e., information that is used to identify a particular lot of assay consumables, which is distinct from lot-specific analysis parameters, which includes that information that is unique to a given lot that may be used by the apparatus, e.g., to conduct an assay with a consumable from that lot or to analyze assay results derived from a consumable from that lot. In one embodiment, if the assay consumable is a multi-well assay plate or a cartridge, the lot-specific analysis parameters may include, but are not limited to, the following: (i) the revision level that determines the schema used to interpret the information; (ii) the consumable type; (iii) the date of manufacture; (iv) the lot number; (v) the date of expiration; (vi) a cross-talk correction matrix, to account for chemical cross-reactivity; (vii) a threshold for assays to be conducted in the consumable and each internal negative control; (viii) a range for each internal positive control; (ix) ranges for each assay to be conducted in the cartridge for the positive control sample; (x) a software checksum to ensure integrity of the data; (xi) in-well (or in-test well) control acceptance ranges; (xii) assay names and/or identifiers; (xiii) information concerning assay quality control, including negative and positive quality control materials that are used to verify the operation of the apparatus and the consumable; (xiv) calibration information such as a master calibration curve; and (xv) number and names of assay calibrators and/or assay calibrator acceptance ranges.

The assay information may include sample information, such as the location of samples within at least one test well of the assay consumable, assay results obtained on the assay consumable for the sample, and the identity of samples that have been and/or will be assay in the assay consumable.

The assay information may also relate to chain of custody, e.g., information regarding the control, transfer and/or analysis of the sample and/or an assay consumable. Chain of custody information may be selected from user identification, sample identification, time and date stamp for an assay, the location of the assay apparatus in a laboratory during the assay, calibration and QC (quality control) status of the assay apparatus during the assay, custody and/or location information for the assay consumable before and after the conduct of the assay, assay results for a given sample, as well as user created free text comments input before, during or after an assay is processed by the apparatus. Still further, chain of custody information may include time, date, manufacturing personnel or processing parameters for one or more steps during the manufacture of the assay consumable, custody, location and/or storage conditions for the assay consumable following manufacture and/or between steps during the manufacture of the assay consumable.

Assay information may also include consumable/test well information, such as consumable type and structure, the location and identity (e.g., the structure, composition, sequence, concentration and/or origin) of assay reagents included within an assay consumable, and the location and identity of assay reagents within an assay test well of the assay consumable.

In addition, the assay information may include assay process information concerning the individual assay parameters that should be applied by the apparatus during the assay. For example, such assay information may include a sequence of steps for a given assay, the identity, concentration and/or quantity of assay reagents that should be used or added during the assay or during a particular step of an assay, e.g., buffers, diluents, and/or calibrators that should be used in that assay. The assay information may also include the type or wavelength of light that should be applied and/or measured by the apparatus during the assay or a particular step of a multi-step assay; the temperature that should be applied by the apparatus during the assay; the incubation time for an assay; and statistical or other analytical methods that should be applied by the apparatus to the raw data collected during the assay.

In an additional embodiment, the information includes consumable/test well/auxiliary well information i.e., information concerning assays previously performed by a apparatus on one or more test or auxiliary wells of the consumable, and information concerning assays to be performed by a apparatus on one or more test or auxiliary wells within the consumable. Therefore, once the assay is conducted by the apparatus, the controller may be used to write the results of the assay to the identifier. Such information includes, but is not limited to raw or analyzed data collected by the apparatus during the assay (wherein analyzed data is data that has been subjected to statistical analysis after collection and raw data is data that has not been subjected to such statistical analysis), a list of test or auxiliary wells within the assay consumable used during a given assay, a schedule of events to be conducted on an assay consumable or a test or auxiliary wells within an assay consumable, a list of those test or auxiliary wells of the assay device that have not be subjected to an assay, assay or apparatus errors that resulted during a given assay or assay step, and combinations thereof.

Moreover, the information comprises data that directly or indirectly controls a component of the assay apparatus, e.g., one or more photodetectors, a light tight enclosure; mechanisms to transport the assay consumables into and out of the apparatus; mechanisms to align and orient the assay consumables with the one or more photodetectors and/or with electrical contacts in the apparatus; additional mechanisms and/or data storage media to track and/or identify assay consumables; one or more sources of electrical energy to induce luminescence; mechanisms to store, stack, move and/or distribute one or more consumables; mechanisms to measure light from a consumable during the assay sequentially, substantially simultaneously or simultaneously from a plurality of test wells of the consumable; and combinations thereof.

Still further, the identifier/controller in the assay apparatus may be used as a security mechanism, e.g., to confirm that the correct assay consumable is being used in the apparatus (referred to herein as "consumable security information"). The assay information may include a digital signature to prove that the consumable was manufactured by the designated vendor. In one embodiment, if an inappropriate assay consumable is present in the apparatus, e.g., a counterfeit consumable or a consumable that is otherwise incompatible with the assay apparatus, the controller will disable the apparatus or a component thereof. In addition or alternatively, the identifier/controller may be used to detect the proper placement of the assay consumable in the apparatus, e.g., the proper orientation of the assay consumable or a portion thereof, in the assay apparatus, such that the controller will disable the apparatus or a component thereof until the assay consumable is placed in the correct orientation. Still further, the identifier/controller in the apparatus may also be used to detect a defect in the assay consumable or test or auxiliary wells and the controller will disable the apparatus, apparatus or a component thereof accordingly. For example, depending on the nature of the defect in the assay consumable, the controller may disallow the use of the assay consumable in its entirety or direct the apparatus to disallow the use of a test or auxiliary well or a set of test or auxiliary wells in the assay consumable. In one embodiment, the apparatus may perform a diagnostic analysis on the assay consumable and/or a test or auxiliary well therein to identify defects therein and the controller will write the results of that diagnostic analysis to the identifier on the consumable. If the consumable is later used in a different apparatus, the results of this diagnostic analysis will be read by the controller and used by the apparatus to adjust the use of that consumable or a test or auxiliary well in that consumable accordingly. In a further embodiment, the assay consumable may be subjected to a quality control process during or after its manufacture and the results of that quality control analysis may be written to the identifier for later use and/or verification by the user of the assay consumable in an assay apparatus.

The assay information may also include authorization information for consumables or test or auxiliary well thereof or biological reagents, such as information regarding whether a particular user has a valid license to use a particular consumable or biological reagent, including the number of times the user is permitted to use the particular consumable or biological reagent in a particular assay and the limitations, if any, on that use, e.g., whether the user's license is for research purposes only. Such information can also include validation information regarding whether a particular consumable or biological reagent has been subject to a recall or has otherwise become unsuitable or unauthorized for use. The recall information and an optional last recall check date and/or timestamp can be written to the identifier.

The assay information may further include information regarding the origin of a biological reagent used in an assay consumable, test or auxiliary well, including for example an identification of an original sample from which it was derived or the number of generations removed it is from an original sample. For example, if an assay reagent used in an assay is an antibody, the assay information may include the identification of the hybridoma from which the antibody was derived, e.g., the ATCC accession number for that hybridoma.

The assay information may additionally include information regarding a consumable, test or auxiliary well or a biological reagent or sample as individual operations are performed on that consumable, test or auxiliary well or biological reagent or sample, for example during manufacture of the consumable, test or auxiliary well or biological reagent or while an assay or step is being performed on the consumable, test or auxiliary well or biological reagent or sample. For example, if an assay consumable includes a plurality of test or auxiliary wells the assay apparatus may perform an assay or step of a multi-step assay on a single test or auxiliary well of the assay consumable. Once that assay or assay step is completed by the assay apparatus, the controller records the results of that assay, e.g., the raw or analyzed data generated during the assay or assay step, to the identifier, and/or the controller records which test or auxiliary well of the assay consumable were used during the assay or assay step and/or which test or auxiliary well of the assay consumable have yet to be used. The assay consumable may be stored for later use and when the user is ready to use another test or auxiliary well of the assay consumable, the controller reads the assay information stored on the identifier of the assay consumable to identify which test or auxiliary well has been used, has yet to be used, and/or the results of those assays. The controller may then instruct the assay apparatus, apparatus or component thereof to conduct an assay or assay step on an unused test or auxiliary well.

In addition, a given assay protocol may require a set of consumables of a particular type. Therefore, if the user inputs a specific type of assay consumable, e.g., a multi-well assay plate, for use in a particular assay protocol, one or more additional assay consumables may be required to carry out that assay protocol in the apparatus, e.g., one or more reagents and a specifically configured auxiliary plate may be required for use with that multi-well assay plate. Each of the required consumables may include a consumable identifier with information concerning the consumable requirements for an assay protocol. When one of the required consumables is input into the assay apparatus and the identifier controller interacts with the consumable identifier for that consumable, the apparatus will take an inventory of the components present in the apparatus and compare the results to the consumable requirements stored to the consumable identifier. If any required consumables are not present or are present in insufficient supply, the apparatus will prompt the user to input the additional required consumables for that assay protocol based on the information stored on the required consumable identifier. If two or more assay consumables are used in the apparatus, the instrument will correctly identify a first assay consumable and any associated consumables based on the consumable requirements stored to the identifiers associated with each consumable. The apparatus will verify that the assay consumable and associated consumables are loaded on the apparatus before the sample is run. In the case where only the first assay consumable is loaded into the apparatus without the corresponding associated consumable, the apparatus will prompt the user to load the associated consumable if the instrument does not identify the associated consumable within the apparatus within a predefined period of time. The apparatus will notify the user if mismatched assay consumables are loaded on the instrument. The apparatus will not run samples if there are no available matched sets of assay consumables (e.g., multi-well assay plates and given reagents for a particular assay). The apparatus will check for assay consumable expiration prior to the start of an assay and the apparatus will alert the user and prevent the use of an expired consumable. The apparatus will not process a sample if the consumables have expired prior to sample aspiration. If a partially used assay consumable is installed into a different instrument, consumable usage will automatically start with the next available unused well.

The identifier may also be used to track the time a given assay consumable is present in the assay apparatus. Therefore, when an assay consumable is inserted into or contacted with an assay apparatus, a timer is initiated in the assay apparatus and the start time is recorded to the identifier. When the assay is initiated by the apparatus on the consumable or a test or auxiliary well within the consumable, the time is also recorded to the identifier. If the instrument, apparatus or a component thereof is shutdown (e.g., by turning the power off), the timer is stopped and that time is recorded to the identifier. Thus, whenever the timer is stopped, the accumulated onboard time is recorded to the identifier.

According to various embodiments, biological samples or reagents that are provided in the carriers described above are licensed separately from apparatus' designed to operate on the biological reagents. In various embodiments the assay apparatus, apparatus or a component thereof is coupled to a network that allows the apparatus to communicate over public and/or private networks with computer apparatus' that are operated by or on behalf of the users, manufacturers and/or licensors of the biological reagents, consumables or apparatus'. In various embodiments, a limited license can provide for the use of licensed biological reagents, consumables or apparatus' for a particular biological analysis on only licensed apparatus'. Accordingly, an apparatus can authenticate a biological reagent, consumable or apparatus based on, for example, a digital signature contained in the identifier associated with a particular consumable, if a particular user has a valid license. In various embodiments, the identifier can also be programmed to provide for a one time use such that biological reagents cannot be refilled for use with the same authentication.

In certain embodiments, when the identifier is read by an apparatus or component thereof that has access to a public or private data network operated by or on behalf of the users, manufacturers and/or licensors of the biological reagents, consumables or apparatus', certain assay information may be communicated to the assay apparatus and read, write or erased locally via the identifier/controller on the assay apparatus. For example, recall and/or license information may be a subset of assay information that is available via the network connections, whereas additional assay information e.g., lot-specific, expiration date, calibration data, consumable specific information, assay domain information, assay results information, consumable security information, or combinations thereof, may be stored locally on the identifier and otherwise unavailable via the network connections on the assay apparatus. In one embodiment, recall, license and/or consumable security information may be available via the network connections on the assay apparatus and the remaining assay information is stored locally on the identifier. The assay apparatus includes apparatus hardware, apparatus firmware, apparatus data acquisition and control software, and method or consumable data. In various embodiments, the apparatus hardware includes electronic control and data processing circuitry, such as a microprocessor or microcontroller, memory, and non-volatile storage. In various embodiments, the apparatus hardware also includes physical devices to manipulate biological reagents such as robotics and sample pumps. In various embodiments, the apparatus firmware includes low-level, computer-readable instructions for carrying out basic operations in connection with the apparatus hardware. In various embodiments, the apparatus firmware includes microprocessor instructions for initializing operations on a microprocessor in the apparatus hardware.

The apparatus data acquisition and control software is higher-level software that interfaces with the apparatus firmware to control the apparatus hardware for more specific operations such as operating a charge coupled device (CCD) to acquire visual luminescence information regarding a particular biological analysis. In various embodiments the data acquisition and control software includes a software-implemented state machine providing, for example, the following states: (i) idle; (ii) running; (iii) paused; and (iv) error. In various embodiments, when the state machine is in the idle state, it can receive an instruction from the general purpose machine to perform a particular data acquisition or apparatus control operation. In various embodiments, the general purpose computer opens a TCP/IP socket connection to the apparatus, determines whether the apparatus is in the idle state and then begins transmitting instructions and/or parameters. In various embodiments, an encrypted TCP/IP connection is established, using, for example, the SSH protocol. The instructions and/or parameters can be in the form of ASCII encoded, human readable consumable and/or method information that defines the behavior of the biological apparatus. In various embodiments, the consumables and/or methods are stored in the form of ASCII text files. In various embodiments, the general purpose computer uses the FTP protocol to transfer the ASCII text files to the apparatus. In various other embodiments the method and/or consumable information is stored in and read from the identifier. The method and/or consumable information can be stored in the form of an ASCII text file in the identifier, but it is understood that the information can be represented in other data formats without departing from the present teachings.

In a further embodiment, the assay apparatus uses a plurality of different assay consumables, e.g., a multi-well assay plates, an auxiliary plate, one or more sample tube racks, and/or containers for assay reagents. A single assay consumable used in the apparatus may include a plurality of consumable identifiers, e.g., a first identifier that includes information that pertains to the entire consumable and one or more additional consumable identifiers of the same or different type that includes information that pertains to a component of that consumable. For example, if the assay consumable is a sample tube rack, the consumable includes an EEPROM, bar code, or RFID with information specific for the entire rack, e.g., lot information and/or lot specific parameters for the rack. The sample tube rack may also include two or more additional identifiers, e.g., a barcode, with information specific for individual samples and/or positions within the rack, e.g., information concerning the sample present at a given position in the rack. In addition, the additional identifier may be used by the apparatus to identify the presence or absence of a sample or reagent in a given position within the rack, e.g., if the additional identifier is obscured and cannot be read by the apparatus, the sample or reagent is present in the rack and if the additional identifier is read by the apparatus, the sample or reagent is not present.

For each type of consumable identifier used by the assay apparatus there is a corresponding identifier controller. For example, if the apparatus uses a multi-well assay plate with an EEPROM identifier and a container for assay reagents with a barcode, then the apparatus will include an EEPROM controller and a barcode controller. Each controller detects and uploads the data stored on a given identifier and the apparatus optionally adjusts one or more assay parameters based on the data uploaded from that identifier. Once the assay is completed, the identifier controller writes information to the identifier concerning that assay or the use of that consumable in the apparatus. The instrument is programmed to reject any consumable that does not have a readable identifier.

The apparatus will prompt the user to scan the reagent identifiers and will record the scanned information. The apparatus will prompt the user to scan the controls, calibrator and reagent identifiers and record the scanned information. The apparatus will persistently track the consumable state so that state can be maintained in the case of a power loss or unexpected shutdown. The apparatus will estimate the volume of fluids in the reagent bottles and it will estimate reagent consumption.

In a specific embodiment, the invention provides an assay apparatus configured to use a multi-well assay plate, an auxiliary plate, and one or more sample tube racks in the conduct of an assay. The assay plate and auxiliary plate have assay plate and auxiliary plate identifiers associated with the respective consumables. Preferably, at least one of the plate identifiers is configured for read/write operation. In one specific example, the auxiliary plate has an EEPROM identifier and the assay plate has a bar code (or visa versa). The sample tube racks also have tube rack identifiers (e.g., bar codes) associated with them to identify the tube rack and, optionally, to identify tube rack positions (as described earlier in the application). The assay apparatus comprises a apparatus adapted to perform the following operations (i) reading tube rack identification and tube position information from a tube rack identifiers associated with the one or more sample tube racks; (ii) reading tube identifiers (e.g., bar codes) associated with sample tubes in the sample tube racks; (iii) reading and writing information to an identifier associated with a first assay consumable selected from an assay plate or an auxiliary plate, preferably an auxiliary plate and iv) reading, and optionally, writing information to an identifier associated with a second different assay consumable selected from an assay plate or an auxiliary plate, preferably an assay plate. The identifier associate with the first assay consumable includes lot and/or assays specific processing and/or analysis parameters and consumable usage information. This information and the tube position information is used to adjust one or more operations performed by the assay apparatus before, during and/or after the conduct of an assay on the multi-well assay plate by the apparatus. The information on the identifier associated with the first consumable is updated by the apparatus to track usage of wells on the first and second consumables and optionally to store information about assay results and the apparatus is configured to erase and/or write information to the identifier. In one embodiment, the assay information included on the identifier is selected from the group consisting of (i) a digital signature to verify manufacturer identify; (ii) lot code of the auxiliary plate or multi-well assay plate; (iii) expiration date of the auxiliary plate or multi-well assay plate; (iv) type of auxiliary plate or multi-well assay plate; (v) serialized identification for the auxiliary plate or multi-well assay plate; and (vi) lot specific parameters for the auxiliary plate or multi-well assay plate. Still further, the lot specific parameters for the multi-well assay plate are selected from the group consisting of (i) in-well control acceptance ranges; (ii) assay names; (iii) assay identifiers; (iv) assay thresholds; (v) number and identity of assay quality controls; (vi) assay quality control acceptance ranges; (vii) calibration information; (viii) number and identity of assay calibrators; (ix) assay calibrator acceptance ranges; (x) chemical cross-talk matrix for the multi-well assay plate; and (xi) combinations thereof. The first consumable identifier may comprise non-volatile memory, e.g., an RFID tag, a bar code, ICC, an EPROM, and EEPROM. In one embodiment, the non-volatile memory is a bar code. The additional consumable identifier comprises non-volatile memory, e.g., an RFID tag, a bar code, ICC, an EPROM, and EEPROM. In one embodiment, the additional consumable identifier is an EEPROM or an RFID.

The invention an apparatus for running assays using the first and second consumables that is configured to carry out a method comprising: i) reading identifiers on the first and second consumables; ii) determining from the lot and assay information that the consumables are valid, non-expired and suitable for being used together; iii) determining which wells are available (e.g., unused) for analyzing samples; iv) analyzing one or more samples using the consumables (optionally, using parameters on the identifiers to adjust one or more operations performed by the apparatus) and v) updating the identifier associated with the first consumable.

One assay procedure using an assay consumable, e.g., a multi-domain multi-well plate and an auxiliary plate, and an assay apparatus would comprise inserting the consumable in the apparatus to allow the identifier controller to interact with the identifier affixed to or associated with the consumable. Alternatively, the consumable packaging includes the identifier affixed thereto or associated therewith and before the consumable is inserted into the apparatus, the identifier associated with the consumable packaging is contacted with the identifier controller. The apparatus may adjust the assay parameters prior to initiating an assay based on the assay information saved to the identifier. Thereafter, the apparatus makes the appropriate electrical, fluidic and/or optical connections to the consumable (making use of electrical, fluidic and/or optical connectors on the consumable and apparatus) and conducts an assay using the consumable. The sample may be introduced into the consumable prior to inserting the consumable in the apparatus. Alternatively, the sample is introduced by a component of the apparatus after the consumable is inserted in the apparatus. The assay may also involve adding one or more assay reagents to the consumable and instructions for adding those various assay reagents may be saved to the identifier and the apparatus adds those reagents to the consumable before or during the assay according to the instructions saved to the assay consumable identifier.

### Examples

### Example 1. One-Step ECL-Based Immunoassay for One or More Target Analytes

A 16 spot 96-well test plate configured for use in ECL-based assays (as shown in Fig. 4(a) and described in the accompanying text) is used in the apparatus described herein and shown, e.g., in Fig. 1. The test plates have integrated screen printed carbon ink electrodes that are suitable for carrying out electrochemiluminescence measurements and a patterned dielectric layer positioned over the working electrode on the bottom of each well that defines 16 "spots" or exposed areas on the working electrode. Various capture antibodies for one or more target analytes of interest are immobilized on the different spots within each well of the test plate. The test plate includes a foil seal covering the wells of the plate.

A 384-well auxiliary plate, as described herein and shown e.g., in Fig. 5(b), includes 96 sets of four auxiliary wells. Each set of auxiliary wells includes at least one well comprising a dried detection reagent comprising one or more detection antibodies and the components of an assay diluent. The detection antibodies bind the one or more target analytes of interest. The detection antibodies are labeled with SULFO-TAG NHS ester (available from Meso Scale Discovery, LLC, a division of Meso Scale Diagnostics, LLC, Gaithersburg, MD), an electrochemiluminescent label based on a sulfonated derivative of ruthenium-tris-bipyridine. A second well of the set of auxiliary wells includes desiccant. The additional two auxiliary wells of each set may, optionally be used for one or more dilution steps in an assay. The auxiliary plate also includes a foil seal covering the wells of the plate.

The auxiliary plate is placed in the auxiliary plate subassembly and the test plate is placed in the inlet plate introduction aperture of the light-tight enclosure of the apparatus as described herein. The seal of a first auxiliary well is pierced by the pipetting arm subassembly and a volume of sample is pipetted by the pipetting arm to the first auxiliary well to reconstitute detection antibodies in the auxiliary well. Thorough mixing is achieved by pipetting the solution by aspiration and dispensing of the solution in the well (also referred to herein as "sip and spit"). The sample is optionally incubated for a predetermined period of time. The seal of the first test well is pierced by the well-wash subassembly (optionally while the detection antibodies are being reconstituted). A volume of the incubated mixture in the first auxiliary well is transferred to the first test well and the mixture was incubated for a predetermined period of time at a preset temperature (depending on the nature of the assay being conducted), with intermittent shaking. The first test well is washed with buffer and filled with an ECL read buffer. The first test well is re-positioned beneath the image detector using the plate carriage. The instrument makes electrical contact to the electrodes in the first test well through contacts on the bottom of the test plate and induces ECL using a linear voltage scan while the image detector images the resulting ECL. Image analysis software is used to quantitate ECL from each spot of the first test well by calculating the total integrated light signal measured over the period of the voltage scan (after correcting for background light levels and detector offset).

The procedure described above is repeated for each set of auxiliary wells and corresponding assay test wells in the plates. To maintain high throughput, interleaved sample processing may be used by the apparatus.

If the assay includes one or more sample dilution steps, a volume of sample is first transferred from the sample rack subassembly to a first dilution well of the auxiliary plate, to which a volume of diluent is also added and mixed with the sample. Optionally, to achieve higher levels of dilution, a volume of diluted sample from the first dilution well is transferred to a second dilution well containing a second volume of diluents. The seal of a first test well is pierced by the well-wash subassembly and a volume of diluted sample is pipetted to the first test well (instead of undiluted sample, as described above). Alternatively, sample dilution may be achieved by addition of diluent to the sample in the test well or first auxiliary well.

### Example 2. Two-Step ECL-Based Immunoassay for One or More Target Analytes

As in Example 1, a 16 spot 96-well multi-well test plate and 384-well auxiliary plate are used in the apparatus described herein and shown e.g., in Fig. 1(a). Various capture antibodies for one or more target analytes of interest are immobilized on the different spots within each well of the test plate. Each set of auxiliary wells includes at least one well comprising dried detection antibodies in an assay diluent. A second well of the set of auxiliary wells includes desiccant. At least two additional wells of the set of auxiliary wells are included for dilution steps, e.g., a serial dilution step.

The auxiliary plate is placed in the auxiliary plate subassembly and the test plate is placed in the inlet plate introduction aperture of the light-tight enclosure of the apparatus as described herein. The seal of a first test well is pierced by the well-wash subassembly and a volume of sample is pipetted to the first test well (optionally, the sample may be diluted as described in Example 1, prior to transfer to the test well). Thorough mixing is achieved by pipetting the solution by repeated aspiration and dispensing of the solution in the well. The sample is incubated for a predetermined period of time at a pre-set temperature (depending on the nature of the assay being conducted), with intermittent shaking. The first test well is washed with wash buffer. While the first test well is prepared, the seal of the first auxiliary well is pierced by the pipetting arm of the pipetting arm subassembly and a volume of diluent is added to the auxiliary well to reconstitute detection antibodies. A volume of reconstituted detection antibodies is added to the first test well, followed by the addition of an ECL read buffer and the mixture is incubated for a predetermined period of time at the preset temperature, with intermittent shaking. The first test well is re-positioned beneath the image detector using the plate carriage and ECL is induced and the assay signal is analyzed and reported as described in Example 1.

The procedure described above is repeated for each set of auxiliary wells and corresponding assay test wells in the plates. To maintain high throughput, interleaved sample processing may be used by the apparatus.

### Example 3. ECL-Based Immunoassays for Upper Respiratory Infection Antigens, Including Subtyping

This example describes a one step immunoassay as in Example 1, but also illustrates additional processing capabilities of the instrumentation: i) simultaneous analysis of samples against multiple panels of target analytes; ii) automated sample preparation on-board the instrument and iii) specific processing steps that have been developed for detection and sub-typing of influenza. In this example, the 16 spot 96-well test plate includes two types of test wells: (i) half (48) of the test wells are designed for detection and typing of influenza (typing wells) and the typing wells include spots with immobilized capture antibodies for influenza nucleoprotein A (NP A) and influenza nucleoprotein B (NP B); and (ii) the other half (48) of the test wells are designed for subtyping of influenza A (subtyping wells) and include spots with immobilized capture antibodies for the H1, H3, H5, H7 and H9 influenza hemagglutinin (HA) subtypes. Both panels also include negative and positive control spots. Similarly, the 4-well sets in the 384 well auxiliary plates are also divided into 48 typing sets and 48 subtyping sets as shown in Table 2 below:

**Table 2**

| Aux. Wells in Typing Set | Auxiliary well (1) | Auxiliary well (2) | Auxiliary well (3) |
|---|---|---|---|
| | Lysis well including | Desiccant | - |
| | (a) a dried reagent comprising a surfactant for lysing influenza virus particles, | | |
| | (b) SULFO-TAG™ labeled detection antibodies against NP A and NP B (and a positive control analyte), | | |
| | (c) positive control analyte and assay diluent components | | |
| Auxiliary wells in Subtyping Set | Acidification well including: | Neutralization well including | Desiccant |
| | (a) a dried reagent comprising a surfactant for lysing influenza virus particles, | (a) a dried reagent comprising a neutralization buffer for raising the pH of samples that have been exposed to the acidification buffer, | |
| | (b) an acidification buffer for inducing optimal presentation of epitopes on the hemagglutinin | | |
| | | (b) SULFO -TAG labeled detection antibodies against H1, H3, H5, H7 and H9 hemagglutinins (and a positive control analyte), | |
| | | (c) positive control analyte and assay diluent components | |

The instrument automates the following assay processing operations. To carry out detection and typing, the instrument transfers sample from a sample tube to a lysis well, mixes the sample in the lysis well to reconstitute the lysis reagent and detection antibodies, transfers the sample to a typing Well, incubates the sample in the well for a pre-determined period of time with intermittent shaking, washes the well with a wash buffer, introduce an ECL read buffer and induces and measures ECL from the well. The process for subtyping analysis is similar, but includes an additional sample preparation step involving acidifying the sample to optimally present the epitopes recognized by the anti-HA antibodies. To carry out detection and typing, the instrument transfers sample from a sample tube to a lysis well, mixes the sample in the lysis well to reconstitute the lysis reagent and detection antibodies, transfers the sample to a typing well, incubates the sample in the well for a pre-determined period of time with intermittent shaking, washes the well with a wash buffer, introduce an ECL read buffer and induces and measures ECL from the well.

The process for subtyping analysis is similar, but includes an additional sample preparation step involving acidifying the sample to optimally present the epitopes recognized by the anti-HA antibodies. To carry out subtyping, the instrument transfers sample from a sample tube to an acidification well, mixes the sample in the acidification well to reconstitute the acidification reagent and incubates the mixture for a predetermined period of time. The instrument then transfers the acidified sample to a neutralization well, mixes the sample in the neutralization well to reconstitute the neutralization reagent and detection antibodies, transfers the sample to a Subtyping Well, incubates the sample in the well for a pre-determined period of time with intermittent shaking, washes the well with a wash buffer, introduce an ECL read buffer and induces and measures ECL from the well.

Table 3 illustrates the scheduling of the assay processing operations in one process block (as defined in the text of the present application). The various operations are performed by different components of the instrument, which are segmented in the table into the pipettor sub-assembly, the well wash subassembly, the ECL detection components and the light tight enclosure (LTE) including the plate translation stage and the enclosure sliding door. The sequence is designed to take advantage of the ability of the different components to operate independently but to make sure they coordinate when required, e.g., when the pipettor, wash station or the ECL detection components need to access specific wells in an assay plate. In this specific example, the incubation in the wells of the assay test plate is allowed to proceed for about 60 minutes, therefore, i) the operations of the pipetting sub-assembly are carried out on a new sample and employ new unused wells of the assay and auxiliary plates and ii) the operations of the well wash and ECL detection components are carried out on a sample that was introduced into a well of the assay plate in a prior process block.

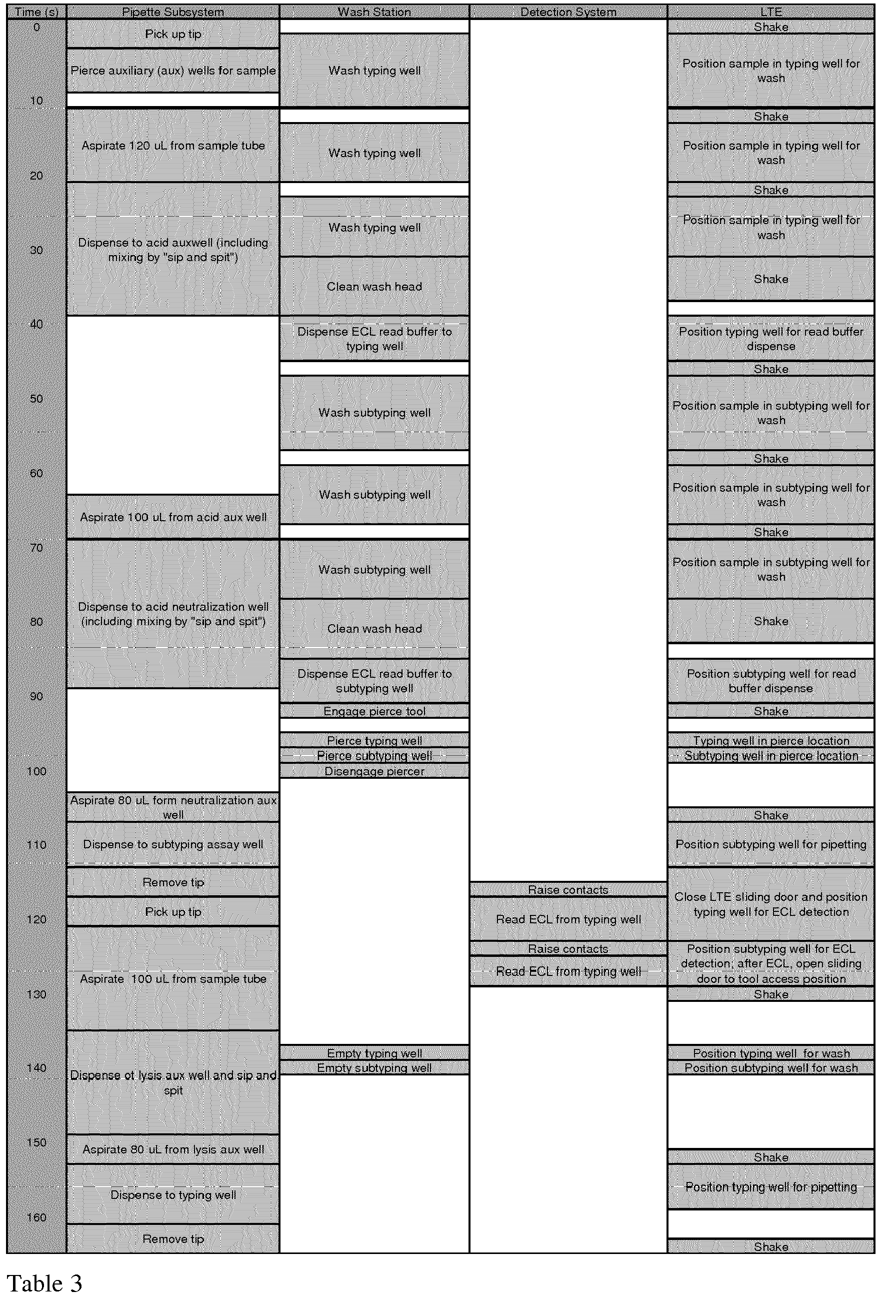

The process block described above is continually repeated as long as there is a sample in process or in the queue for processing, enabling high through-put interleaved operation. Each new sample in the queue is processed using a new set of auxiliary and assay test wells. If there is no sample in the queue or in process that requires a specific operation in the process block, that operation is omitted although the overall timing of the block is maintained.

Patents, patent applications, publications, and test methods cited in this disclosure are incorporated herein by reference in their entirety.

The present invention is not to be limited in scope by the specific embodiments described herein. Indeed, various modifications of the invention in addition to those described herein will become apparent to those skilled in the art from the foregoing description and accompanying drawings. Such modifications are intended to fall within the scope of the claims.

A claim which recites "comprising" allows the inclusion of other elements to be within the scope of the claim; the invention is also described by such claims reciting the transitional phrases "consisting essentially of' (i.e., allowing the inclusion of other elements to be within the scope of the claim if they do not materially affect operation of the invention) or "consisting of' (i.e., allowing only the elements listed in the claim other than impurities or inconsequential activities which are ordinarily associated with the invention) instead of the "comprising" term. Any of these three transitions can be used to claim the invention.

The present invention is further defined by the following items:
1. A kit for conducting luminescence assays in multi-well plates, the kit comprising:
   (a) a multi-well assay test plate comprising a plurality of assay wells for said assay;
   (b) an auxiliary plate comprising a plurality of auxiliary wells, said auxiliary well comprising dry assay reagents for use in said assay with said assay test plate.
2. A kit according to item 1 wherein a well of said test plate comprises a plurality of distinct assay domains, at least two of said domains comprising reagents for measuring different analytes.
3. A kit according to any one of the preceding items wherein said auxiliary plate comprises an identifier comprising assay information used to identify an element selected from the group consisting of (i) said auxiliary plate, (ii) one or more auxiliary wells within said auxiliary plate, (iii) a reagent and/or sample that has been or will be used with said auxiliary plate, (iv) said test plate, (v) one or more wells within said test plate, (vi) a reagent and/or sample that has been or will be used with said test plate, and (vii) combinations thereof.
4. A kit according to item 3 wherein said identifier comprises test plate information identifying a test plate for use with said auxiliary plate.
5. A kit according to item 4 wherein said test plate information comprises test plate lot information.
6. A kit according to item 4 wherein said test plate information comprises a test plate identification number.
7. A kit according to item 1 wherein said assay test plate comprises an identifier comprising assay information used to identify an element selected from the group consisting of (i) said auxiliary plate, (ii) one or more auxiliary wells within said auxiliary plate, (iii) a reagent and/or sample that has been or will be used with said auxiliary plate, (iv) said test plate, (v) one or more wells within said test plate, (vi) a reagent and/or sample that has been or will be used with said test plate, and (vii) combinations thereof.
8. A kit according to item 7 wherein said identifier comprises auxiliary plate information identifying an auxiliary plate for use with said test plate.
9. A kit according to item 8 wherein said auxiliary plate information comprises auxiliary plate lot information.
10. A kit according to item 8 wherein said auxiliary plate information comprises an auxiliary plate identification number.
11. A kit according to item 1 wherein said plurality of auxiliary wells is a multiple of the number of assay wells in said assay test plate.
12. A kit according to item 1 wherein said auxiliary plate comprises twice as many auxiliary wells as assay wells in said assay test plate.
13. A kit according to item 1 wherein said auxiliary plate comprises four times as many auxiliary wells as assay wells in said assay test plate.
14. A kit according to item 1 wherein said assay test plate further comprises a plurality of elements selected from the group consisting of a plate top, plate bottom, working electrodes, counter electrodes, reference electrodes, dielectric materials, electrical connections, dried or liquid assay reagents, and combinations thereof.
15. A kit according to item 1 wherein said auxiliary plate further comprises a set of auxiliary wells, said set comprising adjacent auxiliary wells, wherein said set of auxiliary wells comprises reagents for an assay in a well of said assay test plate.
16. A kit according to item 15 wherein said set comprises four adjacent auxiliary wells.
17. A kit according to item 16 wherein said four adjacent auxiliary wells are arranged in a square.
18. A kit according to item 16 wherein said four adjacent auxiliary wells are arranged in a row.
19. A kit according to item 15 wherein a auxiliary well of said set of auxiliary wells is a dilution well.
20. A kit according to item 15 wherein a auxiliary well of said set of auxiliary wells comprises pre-treated beads.
21. A kit according to item 20 wherein said pre-treated beads are magnetic.
22. A kit according to item 21 wherein said pre-treated beads comprise a coating selected from the group consisting of streptavidin, biotin, and avidin.
23. A kit according to item 22 wherein one or more reagents in said set of auxiliary wells comprise a binding partner of said coating.
24. A kit according to item 15 wherein at least one auxiliary well of said set comprises desiccant and the auxiliary plate comprises a seal.
25. A kit according to item 24 wherein said at least one auxiliary well of said set is connected to an additional auxiliary well of said set via an air passage.
26. A kit according to item 24 wherein said at least one auxiliary well of said set is connected to all auxiliary wells of said set via said air passage.
27. A kit according to item 3 wherein said information is consumable information selected from the group consisting of lot identification information; lot specific analysis parameters, manufacturing process information, raw materials information, expiration date; calibration data; threshold information; the location of individual assay reagents and/or samples within one or more wells and/or auxiliary wells of said auxiliary plate and/or said test plate; Material Safety Data Sheet (MSDS) information, and combinations thereof.
28. A kit according to item 3 wherein said information is sample information selected from the group consisting of the intended location of samples within said one or more wells of the test plate; assay results obtained on said test plate for said sample; identity of samples that have been and/or will be assayed in said test plate; and combinations thereof.
29. A kit according to item 3 wherein said information is chain of custody information.
30. A kit according to item 29 wherein said chain of custody information includes information regarding the control, transfer and/or analysis of a sample.
31. A kit according to item 29 wherein said chain of custody information includes information regarding the control, transfer and/or analysis of a reagent.
32. A kit according to item 29 wherein said information is chain of custody information regarding the control, transfer and/or manufacture of said auxiliary plate and/or test plate.
33. A kit according to item 29 wherein said chain of custody information is selected from the group consisting of user identification; time and date stamp for said assay; location of an assay system using said auxiliary plate and test plate during said assay; calibration and QC status of said assay system during said assay, QC status of said auxiliary plate; QC status of said test plate, custody and/or location information for said auxiliary plate and/or test plate before and after the conduct of said assay; assay results for said sample; and combinations thereof.
34. A kit according to item 29 wherein said information is chain of custody information selected from the group consisting of time, date, manufacturing personnel or processing parameters for one or more steps during the manufacture of said auxiliary plate and/or test plate; custody, location and or storage conditions for said auxiliary plate and/or test plate following manufacture and/or betweens steps during the manufacture of said auxiliary plate and/or test plate; and combinations thereof.
35. A kit according to item 3 wherein said information is auxiliary plate and/or test plate information selected from the group consisting of plate type and structure; location and identity of assay reagents included with said auxiliary plate; location and identify of assay reagents included with said test plate; and combinations thereof.
36. A kit according to item 3 wherein said assay is a multi-step assay and said information is assay process information that relates to a step or steps of said multi-step assay.
37. A kit according to item 3 wherein said information is consumable security information selected from the group consisting of information concerning test plate and/or auxiliary plate authentication; information concerning defects in said test plate, auxiliary plate and/or a test site thereof; and combinations thereof.
38. A kit according to item 1 wherein said test plate comprises capture antibodies for an agent selected from the group consisting of influenza-type A, influenza-type B, RSV, parainfluenza, and adenovirus; and said auxiliary plate comprises detection antibodies for said agent.
39. A kit according to item 38 wherein said auxiliary plate further comprises desiccant.
40. A kit according to item 1 wherein said test plate comprises capture antibodies for an agent selected from the group consisting of influenza-type A, influenza-type B, RSV, parainfluenza, adenovirus, influenza-type A (H1), influenza-type A (H2), influenza-type A (H3), influenza-type A (H5), influenza-type A (H7), influenza-type A (H9); and said auxiliary plate comprises one or more reagents selected from the group consisting of HA acidification buffer, HA neutralization buffer, a detection antibody for said agent, NP, and desiccant.
41. A kit according to item 1 wherein said test plate comprises capture antibodies to a serum biomarker and said auxiliary plate comprises detection antibodies for said biomarker.
42. A kit according to item 41 wherein said auxiliary plate further comprises desiccant.
43. An auxiliary plate comprising a plurality of assay auxiliary well, said auxiliary well comprising dry assay reagents for use in an assay with a corresponding assay test plate.
44. An auxiliary plate according to item 43 wherein said auxiliary plate comprises an identifier comprising assay information used to identify an element selected from the group consisting of (i) said auxiliary plate, (ii) one or more auxiliary wells within said auxiliary plate, (iii) a reagent and/or sample that has been or will be used with said auxiliary plate, (iv) said test plate, (v) one or more wells within said test plate, (vi) a reagent and/or sample that has been or will be used with said test plate, and (vii) combinations thereof.
45. An auxiliary plate according to item 44 wherein said identifier comprises test plate information identifying a test plate for use with said auxiliary plate.
46. An auxiliary plate according to item 45 wherein said test plate information comprises test plate lot information.
47. An auxiliary plate according to item 45 wherein said test plate information comprises a test plate identification number.
48. An auxiliary plate according to item 43 wherein said plurality of auxiliary wells is a multiple of the number of wells in said assay test plate.
49. An auxiliary plate according to item 48 wherein said auxiliary plate comprises twice as many auxiliary wells as wells in said assay test plate.
50. An auxiliary plate according to item 48 wherein said auxiliary plate comprises four times as many auxiliary wells as wells in said assay test plate.
51. An auxiliary plate according to item 43 wherein said auxiliary plate further comprises a set of auxiliary wells, said set comprising adjacent auxiliary wells, wherein said set of auxiliary wells comprises reagents for an assay in a well of said assay test plate.
52. An auxiliary plate according to item 51 wherein said set comprises four adjacent auxiliary wells.
53. An auxiliary plate according to item 52 wherein said four adjacent auxiliary wells are arranged in a square.
54. An auxiliary plate according to item 52 wherein said four adjacent auxiliary wells are arranged in a row.
55. An auxiliary plate according to item 51 wherein a auxiliary well of said set of auxiliary wells is a dilution well.
56. An auxiliary plate according to item 51 wherein a auxiliary well of said set of auxiliary wells comprises pre-treated beads.
57. An auxiliary plate according to item 56 wherein said pre-treated beads are magnetic.
58. An auxiliary plate according to item 56 wherein said pre-treated beads comprise a coating selected from the group consisting of streptavidin, biotin, and avidin.
59. An auxiliary plate according to item 58 wherein one or more reagents in said set of auxiliary wells comprise a binding partner of said coating.
60. An auxiliary plate according to item 51 wherein at least one auxiliary well of said set comprises desiccant and said auxiliary plate comprises a seal.
61. An auxiliary plate according to item 60 wherein said at least one auxiliary well of said set is connected to an additional auxiliary well of said set via an air passage.
62. An auxiliary plate according to item 60 wherein said at least one auxiliary well of said set is connected to all auxiliary wells of said set via said air passage.
63. An auxiliary plate according to item 43 wherein said information is consumable information selected from the group consisting of lot identification information; lot specific analysis parameters, manufacturing process information, raw materials information, expiration date; calibration data; threshold information; the location of individual assay reagents and/or samples within one or more wells and/or auxiliary wells of said auxiliary plate and/or said test plate; Material Safety Data Sheet (MSDS) information, and combinations thereof.
64. An auxiliary plate according to item 43 wherein said information is sample information selected from the group consisting of the intended location of samples within said one or more auxiliary wells of the auxiliary plate; assay results obtained on said test plate for said sample; identity of samples that have been and/or will be assayed in said test plate; and combinations thereof.
65. An auxiliary plate according to item 43 wherein said information is chain of custody information.
66. An auxiliary plate according to item 65 wherein said chain of custody information includes information regarding the control, transfer and/or analysis of a reagent.
67. An auxiliary plate according to item 65 wherein said information is chain of custody information regarding the control, transfer and/or manufacture of said auxiliary plate and/or test plate.
68. An auxiliary plate according to item 65 wherein said chain of custody information is selected from the group consisting of user identification; time and date stamp for said assay; location of an assay system using said auxiliary plate and test plate during said assay; calibration and QC status of said assay system during said assay, QC status of said auxiliary plate; QC status of said test plate, custody and/or location information for said auxiliary plate and/or test plate before and after the conduct of said assay; assay results for said sample; and combinations thereof.
69. An auxiliary plate according to item 65 wherein said information is chain of custody information selected from the group consisting of time, date, manufacturing personnel or processing parameters for one or more steps during the manufacture of said auxiliary plate and/or test plate; custody, location and or storage conditions for said auxiliary plate and/or test plate following manufacture and/or betweens steps during the manufacture of said auxiliary plate and/or test plate; and combinations thereof.
70. An auxiliary plate according to item 43 wherein said information is auxiliary plate and/or test plate information selected from the group consisting of plate type and structure; location and identity of assay reagents included with said auxiliary plate; location and identify of assay reagents included with said test plate; and combinations thereof.
71. An auxiliary plate according to item 43 wherein said assay is a multi-step assay and said information is assay process information that relates to a step or step(s) of said multi-step assay.
72. An auxiliary plate according to item 43 wherein said information is consumable security information selected from the group consisting of information concerning test plate and/or auxiliary plate authentication; information concerning defects in said test plate, auxiliary plate and/or a test well thereof; and combinations thereof.
73. An auxiliary plate according to item 43 wherein said auxiliary plate comprises detection antibodies for an agent selected from the group consisting of influenza-type A, influenza-type B, RSV, Parainfluenza, and adenovirus.
74. An auxiliary plate according to item 73 wherein said auxiliary plate further comprises desiccant.
75. An auxiliary plate according to item 43 wherein said auxiliary plate comprises one or more reagents selected from the group consisting of HA acidification buffer, HA neutralization buffer, NP, desiccant, and a detection antibody for an agent selected from the group consisting of influenza-type A, influenza-type B, RSV, parainfluenza, adenovirus, influenza-type A (H1), influenza-type A (H2), influenza-type A (H3), influenza-type A (H5), influenza-type A (H7), influenza-type A (H9).
76. An auxiliary plate according to item 43 wherein said auxiliary plate comprises detection antibodies to a serum biomarker.
77. An auxiliary plate according to item 76 wherein said auxiliary plate further comprises desiccant.
78. An apparatus for conducting a measurement in a multi-well assay test plate, said apparatus comprising
   (a) a subassembly capable of supporting and translating said test plate to one or more components of said apparatus; and
   (b) an auxiliary plate subassembly, wherein said auxiliary plate comprises a plurality of auxiliary wells comprising dry assay reagents for use in an assay with said test plate.
79. An apparatus according to item 78 further comprising a pipettor subassembly that delivers sample and/or reagent to and from a well of said test plate and/or an auxiliary well of said auxiliary plate.
80. An apparatus according to item 79 wherein said pipettor subassembly comprises a component selected from the group consisting of a pump, a plate piercing probe, a pipetting probe and an ultrasonic sensor.
81. An apparatus according to item 78 wherein said subassembly capable of supporting and translating said test plate comprises a plate introduction aperture and a plate translation stage.
82. An apparatus according to item 81 further comprising a plate stacker adjacent to said plate introduction aperture.
83. An apparatus according to item 82 further comprising a plate elevator comprising a plate lifting platform that can be raised and lowered onto said plate translation stage.
84. An apparatus of item 81 further comprising (i) an input plate introduction aperture comprising an input plate stacker and (ii) an output plate introduction aperture comprising an output plate stacker.
85. An apparatus of any one of items 81 or 84 wherein said plate stacker can accommodate more than one test plate.
86. An apparatus of item 81 wherein said subassembly is a light-tight enclosure and said plate introduction aperture comprises a sliding light-tight door.
87. An apparatus of item 81 wherein said subassembly further comprises a component selected from the group consisting of a thermoelectric heater/cooler, a desiccant chamber, and an identifier controller.
88. An apparatus of item 86 further comprising an imaging system mounted to an imaging aperture in the light-tight enclosure.
89. An apparatus of item 81 wherein said plate translation stage is configured to position a well of a test plate in proximity to one or more components of said subassembly selected from the group consisting of said plate elevator, a well-wash subassembly, and an imaging system.
90. An apparatus of item 89 wherein said well-wash subassembly comprises a seal removal tool, a well-wash head, a wash station, and fluidic connectors to a liquid reagent subassembly.
91. An apparatus of item 90 wherein said well-wash head comprises a pipetting probe and a pipetting translation stage for translating said pipetting probe in a vertical direction.
92. An apparatus of item 91 wherein said pipetting probe comprises a dispensing tube and a plurality of aspiration tubes.
93. An apparatus of item 81 wherein said auxiliary plate subassembly comprises an auxiliary plate introduction aperture and a plate support.
94. An apparatus of item 93 wherein said auxiliary plate subassembly further comprises a housing comprising two or more compartments, each compartment comprising said auxiliary plate introduction aperture and said plate support.
95. An apparatus of item 94 wherein said compartments comprise a component selected from the group consisting of an identifier controller, a thermoelectric heater/cooler, and a desiccant chamber.
96. A method for conducting a measurement in a multi-well assay test plate, said method comprising the steps of:
   (a) dispensing sample and/or reagent into an auxiliary well of an auxiliary plate, said auxiliary plate comprising a plurality of auxiliary wells, said plurality of auxiliary wells comprising dry assay reagents for use in an assay with said test plate; and
   (b) transferring sample and/or reagent from said auxiliary well to a well of a said assay test plate.
97. A method according to item 96 wherein said dispensing step (a) comprises pre-treating said sample and/or reagent in said auxiliary well.
98. A method according to item 97 wherein said transferring step (b) comprises dispensing pre-treated sample and/or reagent from said auxiliary well to said well of said test plate.
99. A method according to item 96 wherein said assay test plate is supported on a plate translation stage and said method comprises translating said test plate via said plate translation stage to one or more components of said apparatus.
100. A method according to item 99 wherein said method further comprises placing said assay test plate through a plate introduction aperture onto a plate stacker adjacent said plate translation stage.
101. A method according to item 100 wherein said method further comprises lowering said assay test plate from said plate stacker to said plate translation stage.
102. A method according to item 101 wherein said method further comprises (i) placing said assay test plate through an input plate introduction aperture comprising an input plate stacker, (ii) lowering said assay test plate from said input plate stacker to said plate translation stage, (iii) translating said assay test plate to one or more components of said apparatus to conduct said measurement, (iv) raising said assay test plate from said plate translation stage to an output plate stacker, and (v) removing said assay test plate from an output plate introduction aperture.
103. A method according to item 96 wherein said method further comprises repeating steps (a) and (b) in an additional auxiliary well of said auxiliary plate and an additional test well of said test plate.
104. A method for conducting a measurement in a multi-well assay test plate, said method comprising the steps of:
   (a) dispensing sample and/or reagent into an auxiliary well of a set of auxiliary wells in an auxiliary plate, said auxiliary plate comprising a plurality of auxiliary wells, said plurality of auxiliary wells comprising dry assay reagents for use in an assay with said test plate;
   (b) pre-treating said sample and/or reagent in one or more auxiliary wells of said set;
   (c) dispensing pre-treated sample and/or reagent from said one or more auxiliary wells of said set to a well of a said assay test plate;
   (d) repeating steps (a)-(c) with an additional sample and reagents in an additional set of auxiliary wells in said auxiliary plate, and an additional well of said test plate;
   (e) ejecting a used auxiliary plate;
   (f) repeating steps (a)-(c) with an additional auxiliary plate; and
   (g) ejecting a used assay test plate.
105. A well-wash subassembly comprising a multi-tube array comprising a central dispensing tube element surrounded by a plurality of aspiration tube elements.
106. A well-wash subassembly according to item 105 wherein said multi-tube array comprises at least two dispensing tube elements at the center of the array.
107. A well-wash subassembly according to item 106 wherein said dispensing tube elements comprise an independent fluid channel for buffers and/or diluents used during an assay.
108. A well-wash subassembly according to item 105 wherein said aspiration tube elements surround the dispensing tube elements and said aspiration tube elements are positioned to align with the outer portions of a well bottom of a multi-well test plate.
109. A well-wash subassembly according to item 105 wherein said aspiration tube elements are independently connected to dedicated fluidic lines.
110. A well-wash subassembly according to item 105 wherein said multi-tube array comprises at least four aspiration tube elements.
111. An apparatus according to item 80 wherein said pipettor subassembly comprises a reflective sensor comprising an infra-red LED and a phototransistor, wherein said LED and phototransistor are positioned in said subassembly to detect the presence or absence of a pipetting tip in said subassembly.

## Claims

1. An auxiliary plate of a kit for conducting luminescence assays in multi-well plates, the auxiliary plate comprising (i) a plurality of auxiliary wells, said auxiliary wells comprising one or more dry assay reagents for use in an assay with a corresponding assay test plate, the corresponding assay test plate comprising a test plate identifier, and (ii) an auxiliary plate identifier comprising assay information that is configured to identify the corresponding test plate, wherein the test plate identifier and auxiliary plate identifier comprise a device selected from the group consisting of an Electrically Erasable Programmable Read Only Memory (EEPROM) and a Radio Frequency Identification Device (RFID), and wherein the test plate identifier and the auxiliary plate identifier store assay information used to identify the test plate and auxiliary plate, respectively, and wherein the device further comprises information that identifies both the test plate and the auxiliary plate for use with each other .

2. An auxiliary plate according to claim 1 wherein said identifier comprises test plate information identifying a test plate for use with said auxiliary plate.

3. An auxiliary plate according to claim 2 wherein said test plate information comprises test plate lot information.

4. An auxiliary plate according to claim 2 wherein said test plate information comprises a test plate identification number.

5. An auxiliary plate according to claim 1 wherein said plurality of auxiliary wells is a multiple of the number of wells in said assay test plate.

6. An auxiliary plate according to claim 5 wherein said auxiliary plate comprises twice as many auxiliary wells as wells in said assay test plate.

7. An auxiliary plate according to claim 5 wherein said auxiliary plate comprises four times as many auxiliary wells as wells in said assay test plate.

8. An auxiliary plate according to claim 1 wherein said auxiliary plate further comprises a set of auxiliary wells, said set comprising adjacent auxiliary wells, wherein said set of auxiliary wells comprises reagents for an assay in a well of said assay test plate.

9. An auxiliary plate according to claim 8 wherein said set comprises four adjacent auxiliary wells.

10. An auxiliary plate according to claim 9 wherein said four adjacent auxiliary wells are arranged in a square.

11. An auxiliary plate according to claim 9 wherein said four adjacent auxiliary wells are arranged in a row.

12. An auxiliary plate according to claim 8 wherein a auxiliary well of said set of auxiliary wells is a dilution well.

13. An auxiliary plate according to claim 8 wherein a auxiliary well of said set of auxiliary wells comprises pre-treated beads.

14. An auxiliary plate according to claim 13 wherein said pre-treated beads are magnetic.

15. An auxiliary plate according to claim 13 wherein said pre-treated beads comprise a coating selected from the group consisting of streptavidin, biotin, and avidin.

16. An auxiliary plate according to claim 15 wherein one or more reagents in said set of auxiliary wells comprise a binding partner of said coating.

17. An auxiliary plate according to claim 8 wherein at least one auxiliary well of said set comprises desiccant and said auxiliary plate comprises a seal.

18. An auxiliary plate according to claim 17 wherein said at least one auxiliary well of said set is connected to an additional auxiliary well of said set via an air passage.

19. An auxiliary plate according to claim 17 wherein said at least one auxiliary well of said set is connected to all auxiliary wells of said set via said air passage.

20. An auxiliary plate according to claim 1 wherein said information is consumable information selected from the group consisting of lot identification information; lot specific analysis parameters, manufacturing process information, raw materials information, expiration date; calibration data; threshold information; the location of individual assay reagents and/or samples within one or more wells and/or auxiliary wells of said auxiliary plate and/or said test plate; Material Safety Data Sheet (MSDS) information, and combinations thereof.

21. An auxiliary plate according to claim 1 wherein said information is sample information selected from the group consisting of the intended location of samples within said one or more auxiliary wells of the auxiliary plate; assay results obtained on said test plate for said sample; identity of samples that have been and/or will be assayed in said test plate; and combinations thereof.

22. An auxiliary plate according to claim 1 wherein said information is chain of custody information.

23. An auxiliary plate according to claim 22 wherein said chain of custody information includes information regarding the control, transfer and/or analysis of a reagent.

24. An auxiliary plate according to claim 22 wherein said information is chain of custody information regarding the control, transfer and/or manufacture of said auxiliary plate and/or test plate.

25. An auxiliary plate according to claim 22 wherein said chain of custody information is selected from the group consisting of user identification; time and date stamp for said assay; location of an assay system using said auxiliary plate and test plate during said assay; calibration and QC status of said assay system during said assay, QC status of said auxiliary plate; QC status of said test plate, custody and/or location information for said auxiliary plate and/or test plate before and after the conduct of said assay; assay results for said sample; and combinations thereof.

26. An auxiliary plate according to claim 22 wherein said information is chain of custody information selected from the group consisting of time, date, manufacturing personnel or processing parameters for one or more steps during the manufacture of said auxiliary plate and/or test plate; custody, location and or storage conditions for said auxiliary plate and/or test plate following manufacture and/or betweens steps during the manufacture of said auxiliary plate and/or test plate; and combinations thereof.

27. An auxiliary plate according to claim 1 wherein said information is auxiliary plate and/or test plate information selected from the group consisting of plate type and structure; location and identity of assay reagents included with said auxiliary plate; location and identify of assay reagents included with said test plate; and combinations thereof.

28. An auxiliary plate according to claim 1 wherein said assay is a multi-step assay and said information is assay process information that relates to a step or step(s) of said multi- step assay.

29. An auxiliary plate according to claim 1 wherein said information is consumable security information selected from the group consisting of information concerning test plate and/or auxiliary plate authentication; information concerning defects in said test plate, auxiliary plate and/or a test well thereof; and combinations thereof.
